# EUROPEAN PATENT APPLICATION

(11) **EP 1 506 965 A1**
(43) Date of publication of application: **16.02.2005**
(21) Application number: 03752916.1
(22) Date of filing: 19.05.2003
(51) Int. Cl.: C07D 333/20

(54) **PROPANOLAMINE DERIVATIVES, PROCESS FOR PREPARATION OF 3-N-METHYLAMINO-1-(2-THIENYL)-1-PROPANOLS AND PROCESS FOR PREPARATION OF PROPANOLAMINE DERIVATIVES**

(30) Priority: 20.05.2002 JP 2002145394
(71) Applicant: Mitsubishi Rayon Co., Ltd., Tokyo 108-8506 (JP)
(72) Inventor: INOUE, Yoshiki, c/o Mitsubishi Rayon Co., Ltd., Yokohama-shi, Kanagawa 230-0053 (JP); MORI, Hiroyuki, c/o Mitsubishi Rayon Co., Ltd., Yokohama-shi, Kanagawa 230-0053 (JP); NOGAMI, Hiroyuki, c/o Mitsubishi Rayon Co., Ltd., Yokohama-shi, Kanagawa 230-0053 (JP); SAITOU, Takayuki, c/o Mitsubishi Rayon Co., Ltd., Otake-shi, Hiroshima 739-0693 (JP); OGURA, Kuniyoshi, c/o Mitsubishi Rayon Co., Ltd., Yokohama-shi, Kanagawa 230-0053 (JP)
(74) Representative: HOFFMANN - EITLE
(86) International application number: PCT/JP2003/006225
(87) International publication number: WO 2003/097632

(57) **Abstract**

The present invention provides means for preparing a racemate or an optically active substance (S- or R-isomer) of 3-N-methylamino-1-(2-thienyl)-1-propanol represented by the following general formula (I): 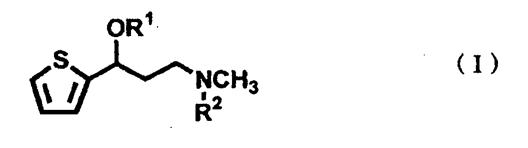 wherein R¹ represents any of a hydrogen atom, a C1-8 acyl group, a substituted or substituted C1-8 alkyloxycarbonyl group and a substituted or substituted phenyloxycarbonyl group and R² represents any of a hydrogen atom, a C1-8 alkyl group, a substituted or substituted benzyl group, a C1-8 acyl group, a substituted or substituted C1-8 alkyloxycarbonyl group and a substituted or substituted phenyloxycarbonyl group, with the exception that R¹ is a hydrogen atom and R² is a methyl group or a hydrogen atom, in a simple manner at low cost and in high yield.

## Description

### TECHNICAL FIELD

The present invention relates to a novel propanolamine derivative which is useful as an intermediate for medicines and agricultural chemicals, and to a process for preparing the same. The present invention also relates to a process for preparing 3-N-methylamino-1-(2-thienyl)-1-propanol using a specific propanolamine derivative including the novel propanolamine derivative.

### BACKGROUND ART

3-N-methylamino-1-(2-thienyl)-1-propanol is a compound which is useful as an intermediate for medicines and agricultural chemicals. Particularly, the S-isomer is known as an essential intermediate of an antidepressant and its production process is disclosed in W. J. Wheeler, F. Kuo, Journal of Labelled Compounds and Radiopharmaceuticals, Vol. XXXVI, No. 3 (1995). This synthesis process is summarized in the following scheme.

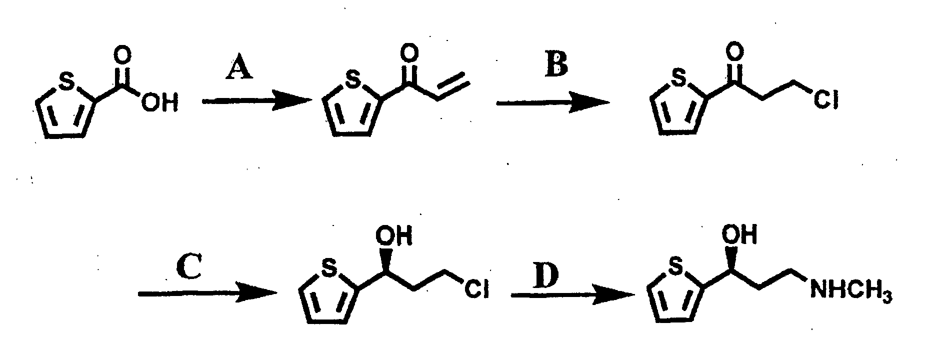

When the process described in Shin Okawara, Journal of the Society of Chemical Industry, Vol. 60, No. 9 (1957) is applied in the preparation of 3-N-methylamino-1-(2-thienyl)-1-propanol, the process by means of dealkylation using N-bromosuccinimide described hereinafter can also be employed. Furthermore, the process by means of dealkylation using bromocyanine described in H. A. Hageman, Org React., 7, 198 (1953) can also be employed.

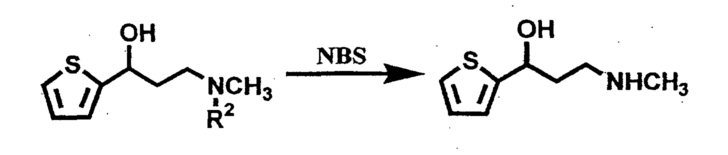

In Jack Deeter, Jeff Frazier, Gilbert Staten, Mike Staszak, Leland Weigel, Tetrahedron Letters, 31 (49), 1990, there is reported the example in which dealkylation is conducted using Troc and then duloxetine is synthesized using Zn. This synthesis process is summarized in the following scheme.

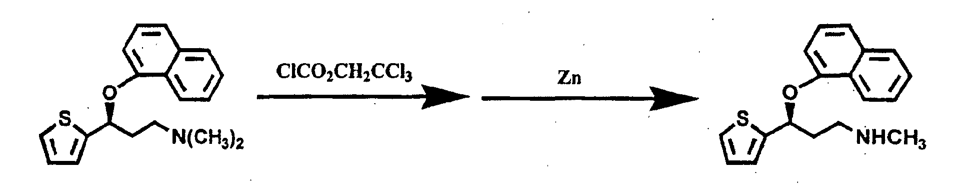

However, the process for preparing a racemate or the R-isomer of 3-N-methylamino-1-(2-thienyl)-1-propanol has never been reported.

The above-mentioned process for preparing the S-isomer had the following problems: 1) the production cost becomes high because of numerous synthesis steps, 2) the starting material is expensive, 3) total yield is low, 4) expensive reducing agent must be used, and 5) column chromatography must be used to purify the product and therefore this process is not an industrial process. The process using N-bromosuccinimide is not a process suited for industrial use because peroxide or ultraviolet irradiation is required and is not suited for used as an industrial process because bromocyanine is a substance having strong toxicity. The process using Troc is not suited for use as an industrial process because of low yield and possibility of the presence of metallic Zn. The process for the synthesis of duloxetine is not an industrially efficient process because a special reducing agent is used and optical purity is low and also the final product is purified in the form of an amine salt.

### DISCLOSURE OF THE INVENTION

Under these circumstances, the present invention has been made and an object thereof is to provide means for preparing a racemate or an optically active substance (S- or R-isomer) of 3-N-methylamino-1-(2-thienyl)-1-propanol in a simple manner at low cost and at high yield.

The present inventors have intensively researched so as to achieve the above object, and thus a novel propanolamine derivative from which 3-N-methylamino-1-(2-thienyl)-1-propanol can be easily derived, and a process for preparing the same have been completed. Also, they have completed a process for preparing 3-N-methylamino-1-(2-thienyl)-1-propanol in the form of a racemate or an optically active substance, using or via a specific propanolamine derivative including a novel propanolamine derivative of the present invention.

The gist of the present invention is "a propanolamine derivative in the form of a racemate or an optically active substance, which is represented by the following general formula (I): 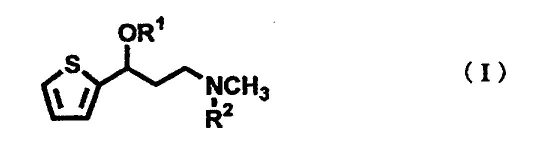 wherein R¹ represents any of a hydrogen atom, an acyl group having 1 to 8 carbon atoms, an alkyloxycarbonyl group having 1 to 8 carbon atoms which may have a substituent and a phenyloxycarbonyl group which may have a substituent, and R² represents any of a hydrogen atom, an alkyl group having 1 to 8 carbon atoms, a benzyl group which may have a substituent, an acyl group having 1 to 8 carbon atoms, an alkyloxycarbonyl group having 1 to 8 carbon atoms which may have a substituent and a phenyloxycarbonyl group which may have a substituent, with the exception that R¹ is a hydrogen atom and R² is a methyl group or a hydrogen atom".

Also the gist of the present invention is "a process for preparing 3-N-methylamino-1-(2-thienyl)-1-propanol in the form of a racemate or an optically active substance, which comprises preparing 3-N-methylamino-1-(2-thienyl)-1-propanol represented by the following general formula (IV): 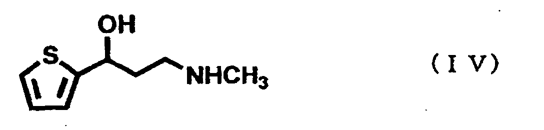 using or via the propanolamine derivative of the present invention".

Also the gist of the present invention is "a process for preparing 3-N-methylamino-1-(2-thienyl)-1-propanol, which comprises preparing 3-N-methylamino-1-(2-thienyl)-1-propanol using or via a propanolamine derivative represented by the following general formula (III): 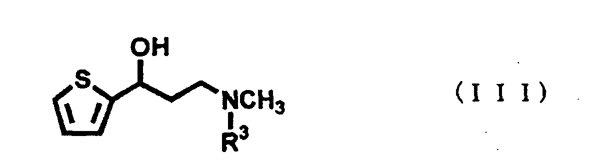 wherein R³ represents an alkyl group having 1 to 8 carbon atoms which may have a substituent or a benzyl group which may have a substituent, or a compound derived from the propanolamine derivative.

### BEST MODE FOR CARRYING OUT THE INVENTION

The process for preparing 3-N-methylamino-1-(2-thienyl)-1-propanol of the present invention is summarized in the following scheme 1.

That is, it is a process in which a dialkylamino alcohol compound (propanolamine derivative represented by the general formula (III) or (VII)) is derived from acetylthiophene and then 3-N-methylamino-1-(2-thienyl)-1-propanol is prepared through the dealkylation step I or I'.

According to this process, 3-N-methylamino-1-(2-thienyl)-2-propanol in any form of racemate, S-isomer and R-isomer can be selectively prepared. In the present specification, 3-N-methylamino-1-(2-thienyl)-1-propanol represented by the general formula (IV) include both the racemate and the optically active substance. Among them, the S-isomer is represented by the general formula (VIII). To distinguish between the production process of the racemate and that of the optically active substance, the compound (IV) is shown as the racemate in the scheme 1.

According to the process of the present invention, the objective compound can be obtained by fewer steps without using a large amount of an expensive reducing agent and also the objective compound can be prepared at low cost, easily within a short time, and at high yield. Particularly, when R³ is a benzyl group which may have a substituent, the objective compound can be prepared at lower cost within a shorter time because the substituent is easily eliminated.

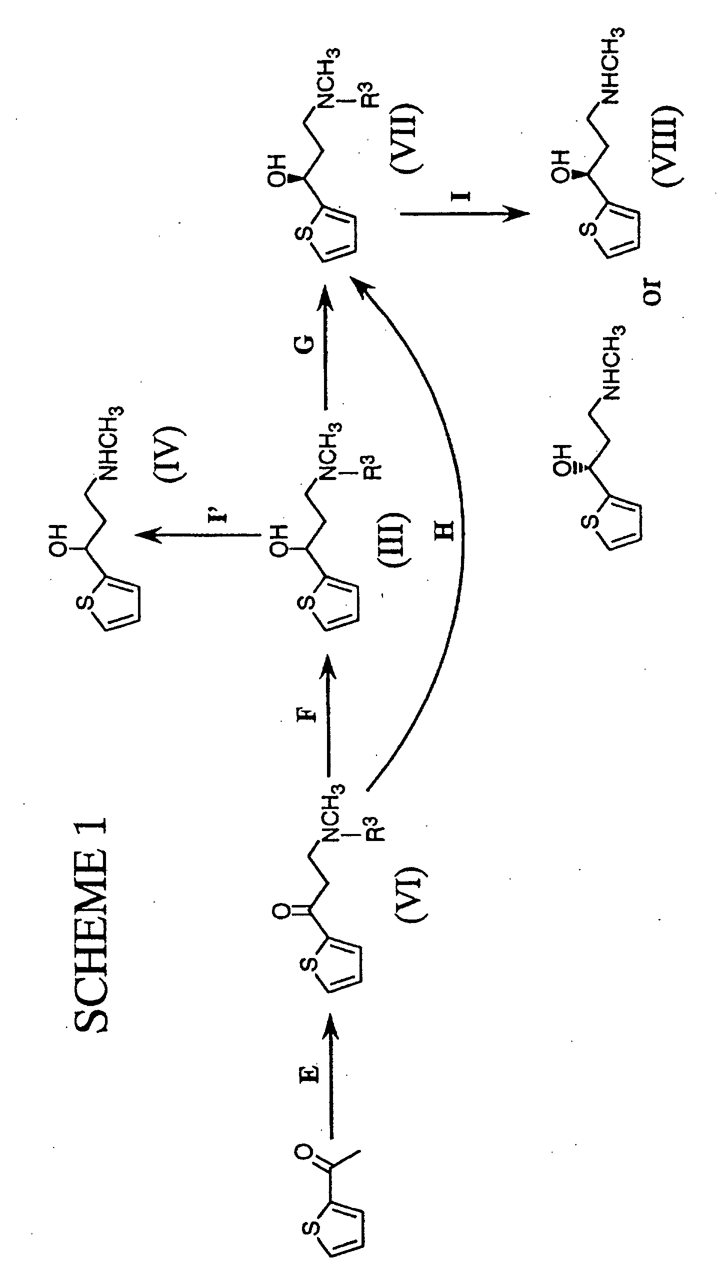

The process of the present invention is particularly
characterized by the dealkylation steps I and I'. As shown in the following scheme 2, these steps can be carried out via various intermediates. These intermediates are characterized in that they can be prepared with high purity and can be isolated without requiring special purification and are also capable of preparing 3-N-methylamino-1-(2-thienyl)-1-propanol represented by the general formula (IV) without isolation. As a matter of course, the intermediates shown in the scheme 2 are illustrative.

Various propanolamine derivatives shown in the schemes 1 and 2, including the intermediates shown in the scheme 2, are novel compounds, and the present invention provides a process for preparing aforementioned 3-N-methylamino-1-(2-thienyl)-1-propanol, these novel propanolamine derivatives and a process for preparing the same.

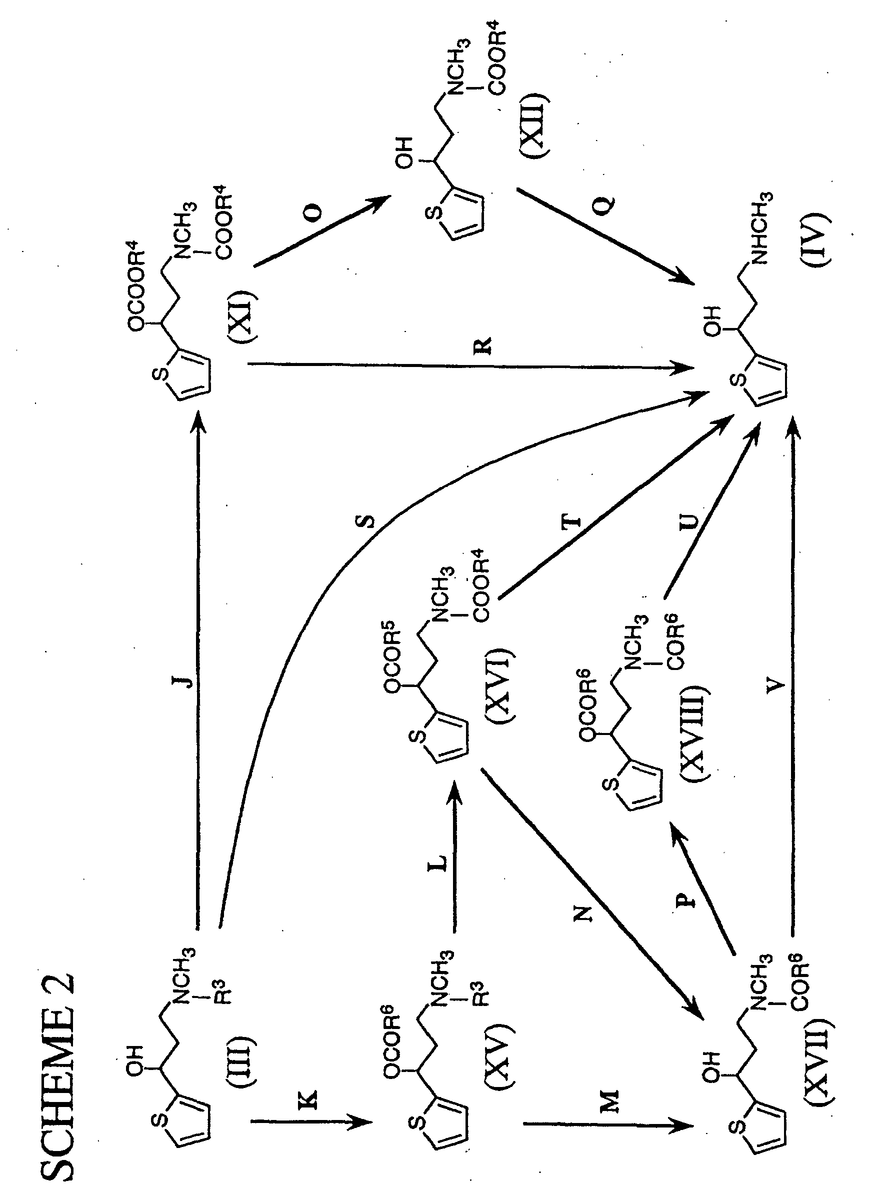

The present invention will now be described in detail. [Propanolamine derivative]

The propanolamine derivative of the present invention is represented by the following general formula (I): 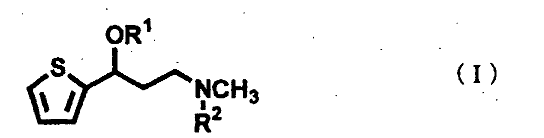 wherein R¹ represents any of a hydrogen atom, an acyl group having 1 to 8 carbon atoms, an alkyloxycarbonyl group having 1 to 8 carbon atoms which may have a substituent and a phenyloxycarbonyl group which may have a substituent, and R² represents any of a hydrogen atom, an alkyl group having 1 to 8 carbon atoms, a benzyl group which may have a substituent, an acyl group having 1 to 8 carbon atoms, an alkyloxycarbonyl group having 1 to 8 carbon atoms which may have a substituent and a phenyloxycarbonyl group which may have a substituent, with the exception that R¹ is a hydrogen atom and R² is a methyl group or a hydrogen atom.

Among these derivatives, a propanolamine derivative in the form of the S-isomer represented by the following general formula (XXIII): 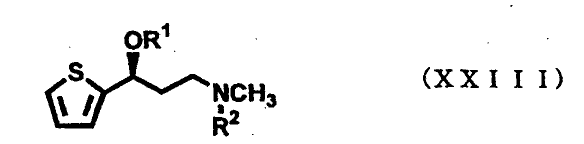 wherein R¹ and R² are as defined above, which is useful as an essential intermediate of an antidepressant and is capable of obtaining (S)-3-N-methylamino-1-(2-thienyl)-1-propanol, is preferable.

Specific examples of the substituent R¹ will now be described.

Examples of the acyl group having 1 to 8 carbon atoms include formyl group, acetyl group, propanoyl group, butanoyl group, a benzoyl group and pivaloyl group. Among these groups, an acetyl group is preferable.

Examples of the alkyloxycarbonyl group having 1 to 8 carbon atoms which may have a substituent include methyloxycarbonyl group, ethyloxycarbonyl group, 2,2,2-trichloroethyloxycarbonyl group, 2-chloroethyloxycarbonyl group, 2,2-dichloroethyloxycarbonyl group, n-propyloxycarbonyl group, iso-propyloxycarbonyl group, n-butyloxycarbonyl group, iso-butyloxycarbonyl group, sec-butyloxycarbonyl group, tert-butyloxycarbonyl group, n-pentyloxycarbonyl group, iso-pentyloxycarbonyl group, sec-pentyloxycarbonyl group, neopentyloxycarbonyl group, 1-methylbutyloxycarbonyl group, 1,2-dimethylpropyloxycarbonyl group, n-hexyloxycarbonyl group, cyclohexyloxycarbonyl group, 1-methylpentyloxycarbonyl group and 2-ethylbutyloxycarbonyl group. Among these groups, a 2,2,2-trichloroethyloxycarbonyl group, an iso-propyloxycarbonyl group and an iso-butyloxycarbonyl group are preferable.

Examples of the phenyloxycarbonyl group which may have a substituent include phenyloxycarbonyl group, chlorophenyloxycarbonyl group and tolyloxycarbonyl group. Among these groups, a phenyloxycarbonyl group is preferable.

Specific examples of the substituent R² will now be described.

Examples of the alkyl group having 1 to 8 carbon atoms which may have a substituent include methyl group, ethyl group, trichloroethyl group, monochloroethyl group, dichloroethyl group, n-propyl group, iso-propyl group, n-butyl group, iso-butyl group, sec-butyl group, tert-butyl group, n-pentyl group, iso-pentyl group, sec-pentyl group, neopentyl group, 1-methylbutyl group, 1,2-dimethylpropyl group, n-hexyl group, 1-methylpentyl group, cyclohexyl group and 2-ethylbutyl group. Among these groups, a methyl group is preferable, with the exception that R¹ is a hydrogen atom and R² is a methyl group.

Examples of the benzyl group which may have a substituent include benzyl group, 3,4-dimethoxybenzyl group and 2-nitrobenzyl group. Among these groups, a benzyl group is preferable.

Examples of the acyl group having 1 to 8 carbon atoms, the alkyloxycarbonyl group having 1 to 8 carbon atoms which may have a substituent and the phenyloxycarbonyl group which may have a substituent include those which are exemplified as for the substituent R¹.

Specific examples of the propanolamine derivative of the present invention, which is represented by the general formula (I) or (XXIII), include ethyl (S)-N-[3-ethyloxycarbonyloxy-3-(2-thienyl)propyl]-N-methylcarbamate, ethyl (RS)-N-[3-ethyloxycarbonyloxy-3-(2-thienyl)propyl]-N-methylcarbamate, ethyl (R)-N-[3-ethyloxycarbonyloxy-3-(2-thienyl)propyl]-N-methylcarbamate, methyl (S)-N-[3-methyloxycarbonyloxy-3-(2-thienyl)propyl]-N-methylcarbamate, methyl (RS)-N-[3-methyloxycarbonyloxy-3-(2-thienyl)propyl]-N-methylcarbamate, methyl (R)-N-[3-methyloxycarbonyloxy-3-(2-thienyl)propyl]-N-methylcarbamate, 2',2',2'-trichloroethyl (S)-N-methyl-N-[3-(2'',2'',2''-trichloroethyloxycarbonyloxy-3-(2-thienyl)propyl)carbamate, 2',2',2'-trichloroethyl (RS)-N-methyl-N-[3-(2'',2'',2''-trichloroethyloxycarbonyloxy)-3-(2-thienyl)propyl]carbamate, 2',2',2'-trichloroethyl (R)-N-methyl-N-[3-(2'',2'',2''-trichloroethyloxycarbonyloxy)-3-(2-thienyl)propyl]carbamate, phenyl (S)-N-methyl-N-[3-phenyloxycarbonyloxy-3-(2-thienyl)propyl]carbamate, phenyl (RS)-N-methyl-N-[3-phenyloxycarbonyloxy-3-(2-thienyl)propyl]carbamate, phenyl(R)-N-methyl-N-[3-phenyloxycarbonyloxy-3-(2-thienyl)propyl]carbamate, iso-propyl (S)-N-[3-iso-propyloxycarbonyloxy-3-(2-thienyl)propyl]-N-methylcarbamate, iso-butyl (S)-N-[3-iso-butyloxycarbonyloxy-3-(2-thienyl)propyl]-N-methylcarbamate, (S)-N,N-dimethyl-N-[3-phenyloxycarbonyloxy-3-(2-thienyl)propyl] amine, (S)-N,N-dimethyl-N-[3-iso-propyloxycarbonyloxy-3-(2-thienyl)propyl]amine, (RS)-N-[3-ethoxycarbonyloxy-3-(2-thienyl)propyl]-N,N-dimethylamine, (R)-N-[3-ethoxycarbonyloxy-3-(2-thienyl)propyl]-N,N-dimethylamine, (S)-N-[3-iso-butyloxycarbonyloxy-3-(2-thienyl)propyl]-N-benzyl-N-methylamine, (S)-N-[3-iso-butyloxycarbonyloxy-3-(2-thienyl)propyl]-N,N-dimethylamine, (RS)-N-[3-ethoxycarbonyloxy-3-(2-thienyl)propyl]-N-benzyl-N-methylamine, (R)-N-[3-ethoxycarbonyloxy-3-(2-thienyl)propyl]-N-benzyl-N-methylamine, (S)-N-[3-phenyloxycarbonyloxy-3-(2-thienyl)propyl]-N-benzyl-N-methylamine, (RS)-N-[3-phenyloxycarbonyloxy-3-(2-thienyl)propyl]-N-benzyl-N-methylamine, (S)-N-[3-hydroxy-3-(2-thienyl)propyl]-N-methylacetamide, (RS)-N-[3-hydroxy-3-(2-thienyl)propyl]-N-methylacetamide, (R)-N-[3-hydroxy-3-(2-thienyl)propyl]-N-methylacetamide, (S)-N-[3-hydroxy-3-(2-thienyl)propyl]-N-methylbutaneamide, (S)-N-[3-hydroxy-3-(2-thienyl)propyl]-N-methylbenzamide, (RS)-N-[3-hydroxy-3-(2-thienyl)propyl]-N-methylbenzamide, iso-propyl (S)-N-[3-hydroxy-3-(2-thienyl)propyl]-N-methylcarbamate, iso-butyl (S)-N-[3-hydroxy-3-(2-thienyl)propyl]-N-methylcarbamate, iso-propyl (RS)-N-[3-hydroxy-3-(2-thienyl)propyl]-N-methylcarbamate, iso-butyl (RS)-N-[3-hydroxy-3-(2-thienyl)propyl]-N-methylcarbamate, ethyl (S)-N-[3-hydroxy-3-(2-thienyl)propyl]-N-methylcarbamate, ethyl (RS)-N-[3-hydroxy-3-(2-thienyl)propyl]-N-methylcarbamate, methyl (RS)-N-[3-hydroxy-3-(2-thienyl)propyl]-N-methylcarbamate, methyl (S)-N-[3-hydroxy-3-(2-thienyl)propyl]-N-methylcarbamate, phenyl (S)-N-[3-hydroxy-3-(2-thienyl)propyl]-N-methylcarbamate, phenyl (RS)-N-[3-hydroxy-3-(2-thienyl)propyl]-N-methylcarbamate, 2',2',2'-trichloroethyl (S)-N-[3-hydroxy-3-(2-thienyl)propyl]-N-methylcarbamate, 2',2',2'-trichloroethyl (RS)-N-[3-hydroxy-3-(2-thienyl)propyl]-N-methylcarbamate, iso-propyl (S)-N-[3-acetoxy-3-(2-thienyl)propyl]-N-methylcarbamate, iso-propyl (RS)-N-[3-acetoxy-3-(2-thienyl)propyl]-N-methylcarbamate, iso-butyl (S)-N-[3-acetoxy-3-(2-thienyl)propyl]-N-methylcarbamate, iso-butyl (RS)-N-[3-acetoxy-3-(2-thienyl)propyl]-N-methylcarbamate, cyclohexyl (S)-N-[3-acetoxy-3-(2-thienyl)propyl]-N-methylcarbamate, cyclohexyl (RS)-N-[3-acetoxy-3-(2-thienyl)propyl]-N-methylcarbamate, ethyl (S)-N-[3-acetoxy-3-(2-thienyl)propyl]-N-methylcarbamate, ethyl (RS)-N-[3-acetoxy-3-(2-thienyl)propyl]-N-methylcarbamate, methyl (S)-N-[3-acetoxy-3-(2-thienyl)propyl]-N-methylcarbamate, methyl (RS)-N-[3-acetoxy-3-(2-thienyl)propyl]-N-methylcarbamate, phenyl (S)-N-[3-acetoxy-3-(2-thienyl)propyl]-N-methylcarbamate, phenyl (RS)-N-[3-acetoxy-3-(2-thienyl)propyl]-N-methylcarbamate, phenyl (S)-N-[3-benzoyloxy-3-(2-thienyl)propyl]-N-methylcarbamate, (S)-N-[3-acetoxy-3-(2-thienyl)propyl]-N-methylacetamide, (RS)-N-[3-acetoxy-3-(2-thienyl)propyl]-N-methylacetamide, (R)-N-[3-acetoxy-3-(2-thienyl)propyl]-N-methylacetamide, (S)-N-[3-butanoyl-3-(2-thienyl)propyl]-N-methylbutaneamide, (RS)-N-[3-butanoyl-3-(2-thienyl)propyl]-N-methylbutaneamide, (S)-N-[3-benzoyloxy-3-(2-thienyl)propyl]-N-methylacetamide, (S)-N-[3-benzoyloxy-3-(2-thienyl)propyl]-N-methylbenzamide, (RS)-N-[3-benzoyloxy-3-(2-thienyl)propyl]-N-methylbenzamide, (S)-N-[3-acetoxy-3-(2-thienyl)propyl]-N-methyl-N-benzylamine, (RS)-N-[3-acetoxy-3-(2-thienyl)propyl]-N-methyl-N-benzylamine, (R)-N-[3-acetoxy-3-(2-thienyl)propyl]-N-methyl-N-benzylamine, (S)-N-[3-acetoxy-3-(2-thienyl)propyl]-N-dimethylamine, (RS)-N-[3-acetoxy-3-(2-thienyl)propyl]-N-dimethylamine, (R)-N-[3-acetoxy-3-(2-thienyl)propyl]-N-dimethylamine, phenyl (S)-N-[3-acetoxy-3-(2-thienyl)propyl]-N-methylcarbamate, phenyl(R)-N-[3-acetoxy-3-(2-thienyl)propyl]-N-methylcarbamate, phenyl (RS)-N-[3-acetoxy-3-(2-thienyl)propyl]-N-methylcarbamate, iso-propyl (S)-N-[3-acetoxy-3-(2-thienyl)propyl]-N-methylcarbamate, iso-propyl (RS)-N-[3-acetoxy-3-(2-thienyl)propyl]-N-methylcarbamate, iso-butyl (S)-N-[3-acetoxy-3-(2-thienyl)propyl]-N-methylcarbamate, iso-butyl (RS)-N-[3-acetoxy-3-(2-thienyl)propyl]-N-methylcarbamate, cyclohexyl (S)-N-[3-acetoxy-3-(2-thienyl)propyl]-N-methylcarbamate, cyclohexyl (RS)-N-[3-acetoxy-3-(2-thienyl)propyl]-N-methylcarbamate, ethyl (S)-N-[3-acetoxy-3-(2-thienyl)propyl]-N-methylcarbamate, ethyl (R)-N-[3-acetoxy-3-(2-thienyl)propyl]-N-methylcarbamate, ethyl (RS)-N-[3-acetoxy-3-(2-thienyl)propyl]-N-methylcarbamate, (S)-N-benzyl-N-methylamino-1(2-thienyl)-1-propanol, (RS)-N-benzylmethylamino-1(2-thienyl)-1-propanol, (R)-N-benzylmethylamino-1(2-thienyl)-1-propanol, (S)-N-[3-acetoxy-3-(2-thienyl)propyl]-N-methylacetamide, (R)-N-[3-acetoxy-3-(2-thienyl)propyl]-N-methylacetamide and (RS)-N-[3-acetoxy-3-(2-thienyl)propyl]-N-methylacetamide.

Among these derivatives, preferable derivatives are ethyl (S)-N-[3-ethyloxycarbonyloxy-3-(2-thienyl)propyl]-N-methylcarbamate, ethyl (RS)-N-[3-ethyloxycarbonyloxy-3-(2-thienyl)propyl]-N-methylcarbamate, methyl (S)-N-[3-methyloxycarbonyloxy-3-(2-thienyl)propyl]-N-methylcarbamate, methyl (RS)-N-[3-methyloxycarbonyloxy-3-(2-thienyl)propyl]-N-methylcarbamate, 2',2',2'-trichloroethyl (S)-N-methyl-N-[3-(2'',2'',2''-trichloroethyloxycarbonyloxy-3-(2-thienyl)propyl)carbamate, 2',2',2'-trichloroethyl (RS)-N-methyl-N-[3-(2'',2'',2''-trichloroethyloxycarbonyloxy)-3-(2-thienyl)propyl]carbamate, phenyl (S)-N-methyl-N-[3-phenyloxycarbonyloxy-3-(2-thienyl)propyl]carbamate, phenyl (RS)-N-methyl-N-[3-phenyloxycarbonyloxy-3-(2-thienyl)propyl]carbamate, iso-propyl (S)-N-[3-iso-propyloxycarbonyloxy-3-(2-thienyl)propyl]-N-methylcarbamate, iso-butyl (S)-N-[3-iso-butyloxycarbonyloxy-3-(2-thienyl)propyl]-N-methylcarbamate, iso-propyl (RS)-N-[3-iso-propyloxycarbonyloxy-3-(2-thienyl)propyl]-N-methylcarbamate, iso-butyl (RS)-N-[3-iso-butyloxycarbonyloxy-3-(2-thienyl)propyl]-N-methylcarbamate, (RS)-N,N-dimethyl-N-[3-phenyloxycarbonyloxy-3-(2-thienyl)propyl] amine, (S)-N,N-dimethyl-N-[3-phenyloxycarbonyloxy-3-(2-thienyl)propyl] amine, (RS)-N,N-dimethyl-N-[3-iso-propyloxycarbonyloxy-3-(2-thienyl)propyl]amine, (S)-N,N-dimethyl-N-[3-iso-propyloxycarbonyloxy-3-(2-thienyl)propyl]amine, (RS)-N,N-dimethyl-N-[3-iso-butyloxycarbonyloxy-3-(2-thienyl)propyl]amine, (S)-N,N-dimethyl-N-[3-iso-butyloxycarbonyloxy-3-(2-thienyl)propyl]amine, (RS)-N-[3-ethoxycarbonyloxy-3-(2-thienyl)propyl]-N,N-dimethylamine, (S)-N-[3-ethoxycarbonyloxy-3-(2-thienyl)propyl]-N,N-dimethylamine, (S)-N-[3-iso-butyloxycarbonyloxy-3-(2-thienyl)propyl]-N-benzyl-N-methylamine, (S)-N-[3-iso-butyloxycarbonyloxy-3-(2-thienyl)propyl]-N,N-dimethylamine, (S)-N-[3-hydroxy-3-(2-thienyl)propyl]-N-methylacetamide, (RS)-N-[3-hydroxy-3-(2-thienyl)propyl]-N-methylacetamide, iso-propyl (S)-N-[3-hydroxy-3-(2-thienyl)propyl]-N-methylcarbamate, iso-butyl (S)-N-[3-hydroxy-3-(2-thienyl)propyl]-N-methylcarbamate, iso-propyl (RS)-N-[3-hydroxy-3-(2-thienyl)propyl]-N-methylcarbamate, iso-butyl (RS)-N-[3-hydroxy-3-(2-thienyl)propyl]-N-methylcarbamate, ethyl (S)-N-[3-hydroxy-3-(2-thienyl)propyl]-N-methylcarbamate, ethyl (RS)-N-[3-hydroxy-3-(2-thienyl)propyl]-N-methylcarbamate, 2',2',2'-trichloroethyl (S)-N-[3-hydroxy-3-(2-thienyl)propyl]-N-methylcarbamate, 2',2',2'-trichloroethyl (RS)-N-[3-hydroxy-3-(2-thienyl)propyl]-N-methylcarbamate, phenyl (S)-N-[3-hydroxy-3-(2-thienyl)propyl]-N-methylcarbamate, phenyl (RS)-N-[3-hydroxy-3-(2-thienyl)propyl]-N-methylcarbamate, (S)-N-[3-acetoxy-3-(2-thienyl)propyl]-N-methyl-N-benzylamine, (RS)-N-[3-acetoxy-3-(2-thienyl)propyl]-N-methyl-N-benzylamine, (RS)-N-[3-acetoxy-3-(2-thienyl)propyl]-N-dimethylamine, (S)-N-[3-acetoxy-3-(2-thienyl)propyl]-N-dimethylamine, phenyl (S)-N-[3-acetoxy-3-(2-thienyl)propyl]-N-methylcarbamate, phenyl (RS)-N-[3-acetoxy-3-(2-thienyl)propyl]-N-methylcarbamate, iso-propyl (S)-N-[3-acetoxy-3-(2-thienyl)propyl]-N-methylcarbamate, iso-propyl (RS)-N-[3-acetoxy-3-(2-thienyl)propyl]-N-methylcarbamate, iso-butyl (S)-N-[3-acetoxy-3-(2-thienyl)propyl]-N-methylcarbamate, iso-butyl (RS)-N-[3-acetoxy-3-(2-thienyl)propyl]-N-methylcarbamate, (S)-N-benzyl-N-methylamino-1(2-thienyl)1-1-propanol, (RS)-N-benzylmethylamino-1(2-thienyl)-1-propanol, (S)-N-[3-acetoxy-3-(2-thienyl)propyl]-N-methylacetamide and (RS)-N-[3-acetoxy-3-(2-thienyl)propyl]-N-methylacetamide.

### [Process for preparing propanolamine derivative and 3-N-methylamino-1-(2-thienyl)-1-propanol]

The process for preparing the propanolamine derivative of the present invention, and the process for preparing 3-N-methylamino-1-(2-thienyl)-1-propanol in the form of a racemate or an optically active substance via a specific propanolamine derivative including the propanolamine derivative will now be described.

As described previously, the process of the present invention is summarized in the schemes 1 and 2 and, as shown in the schemes, various synthetic routes exist, and thus the objective compound can be prepared through a desired route. Although acetylthiophene is used as a starting material in the schemes, various derivatives obtained therefrom may be used as a starting material.

In the schemes 1 and 2, the substituent R³ represents an alkyl group having 1 to 8 carbon atoms which may have a substituent or a benzyl group which may have a substituent. The steps E, F and G when the substituent R³ is a methyl group are described in Tetrahedron Letters, 31 (49), 7101-04 (1990). The step H when the substituent R³ is a methyl group is described in Angew. Chem. Int. Ed. 40, 40-73 (2001). Therefore, in the steps of the present invention, the steps E, F, G and H are novel when the substituent R³ is a functional group other than a methyl group, particularly the substituent R³ is a benzyl group which may have a substituent, while other steps are novel regardless of the structure of the substituent R³.

### (Step E)

The step E is the step of preparing a ketone compound represented by the general formula (VI) by condensing acetylthiophene and a dialkylamine (N-alkylmethylamine having 1 to 8 carbon atoms which may have a substituent, or N-benzylmethylamine which may have a substituent) represented by the following general formula (V):

**HNCH**_{**3**}**R**^{**3**} (V)

wherein R³ represents an alkyl group having 1 to 8 carbon atoms which may have a substituent or a benzyl group which may have a substituent, and formalin (formalin source such as paraform or trioxane can also be used) in the presence of an acid catalyst (under acidic conditions).

Examples of the benzyl group which may have a substituent include benzyl group, 3,4-dimethoxybenzyl group and 2-nitrobenzyl group.

Specific examples of N-alkylmethylamine having 1 to 8 carbon atoms which may have a substituent or N-benzylmethylamine which may have a substituent used in the step E include N-ethylmethylamine, N-methyltrichloroethylamine, N-methylmonochloroethylamine, N-dichloroethylmethylamine, N-n-propylmethylamine, N-iso-propylmethylamine, N-n-butylmethylamine, N-iso-butylmethylamine, N-sec-butylmethylamine, N-tert-butylmethylamine, N-n-pentylmethylamine, N-iso-pentylmethylamine, N-sec-pentylmethylamine, N-neopentylmethylamine, N-1-methylbutylmethylamine, N-1,2-dimethylpropylmethylamine, N-n-hexylmethylamine, N-1-methylpentylmethylamine, N-2-ethylbutylmethylamine, N-benzylmethylamine, N-chlorobenzylmethylamine, and their mineral acid salts with hydrochloric acid or the like.

The solvent used in the step E is not specifically limited as long as it does not exert an adverse influence on the reaction, and examples thereof include amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; nitriles such as acetonitrile; ethers such as diisopropyl ether, tetrahydrofuran, 1,4-dioxane, 1,2-dimethoxyethane and anisole; and alcohols such as methanol, ethanol, propanol, ethylene glycol and propylene glycol. These solvents can be used alone or in combination.

The reaction temperature is preferably within a range from 0 to 150°C, and more preferably from 30 to 100°C.

### (Step F)

The step F is the step of reducing the ketone compound represented by the general formula (VI) obtained in the step E to obtain an alcohol compound represented by the general formula (III).

Examples of the reducing agent used in the step F include sodium borohydride, lithium aluminum hydride and sodium dihydrobis(2-methoxyethoxy)aluminum. Among these reducing agents, sodium borohydride is particularly preferable.

As the reaction solvent, an organic solvent, which is inert to the reaction, can be used, and examples thereof include aromatic hydrocarbons such as toluene and xylene; halogenated hydrocarbons such as methylene chloride and chloroform; ethers such as diethyl ether, diisopropyl ether, tetrahydrofuran, 1,4-dioxane and t-butyl methyl ether; ester solvents such as ethyl acetate and methyl acetate; nitrile solvents such as acetonitrile; and alcohols such as methanol, ethanol, propanol, ethylene glycol and propylene glycol. These solvents can be used alone or in combination.

The reaction temperature is preferably from -20 to 100°C, and more preferably from 0 to 40°C. The reaction time is preferably within 12 hours, and more preferably from 0.5 to 6 hours.

### (Step G)

The step G is the step of obtaining a diastereomer salt of the propanolamine derivative represented by the general formula (III) obtained in the step F with an optically active organic acid, and optically resolving the diastereomer salt to obtain an optically active amino alcohol. Consequently, a propanolamine derivative in the form of the S- or R-isomer can be obtained. The scheme 1 illustrates the case where the S-isomer represented by the general formula (VII) is obtained.

Examples of the optically active organic acid used in the step G include optically active carboxylic acid, optically active sulfonic acid and optically active phosphonic acid, which are represented by the following general formula (XXI): 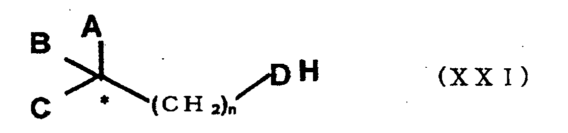 wherein D represents any of COO⁻, SO₃⁻ and PO₃H⁻, A, B, C each independently represents any of a hydrogen atom, a substituted or unsubstituted linear or branched alkyl group having 1 to 10 carbon atoms, a halogen atom, an alkoxy group, a hydroxyl group, a nitro group, a carboxyl group, a substituted or unsubstituted phenyl group and a naphthyl group, the substituent of the alkyl group, phenyl group and naphthyl group represents any of a linear or branched alkyl group having 1 to 10 carbon atoms, a halogen atom, an alkoxy group, a hydroxyl group, a nitro group, a carboxyl group and a sulfonate group, A, B, C and (CH₂)ₙ-DH are different substituents groups, n represents 1 or 0, and the symbol * represents an asymmetric carbon. Specific examples of the optically active carboxylic acid include optically active hydroxycarboxylic acids such as tartaric acid, malic acid, lactic acid, mandelic acid, dibenzoyltartaric acid, citramalic acid, phenyllactic acid and 1,4-benzodioxane-2-carboxylic acid, and derivatives thereof, 2-bromopropionic acid, γ-carboxy-γ-butyrolactone, 2-chlorobutanoic acid, 2-methylhexanoic acid, 2-methyldecanoic acid, 2-methylbutanoic acid, methyloxyacetic acid, tetrahydrofuran acid, 2-phenylbutanoic acid, 2-phenylpropionic acid, 2-phenylsuccinic acid, optically active N-substituted amino acid, pyroglutamic acid and camphoric acid.

Examples of the optically active sulfonic acid include 10-camphorsulfonic acid, phenylethanesulfonic acid, α-bromocamphor-n-sulfonic acid and 3-endobromocamphor-8-sulfonic acid, and examples of the optically active phosphonic acid include 1-amino-2-methylpropylphosphonic acid.

Among optically active carboxylic acids, particularly preferable optically active carboxylic acid is an optically active 2-aryl-2-substituted acetic acid represented by the general formula (XXIV): 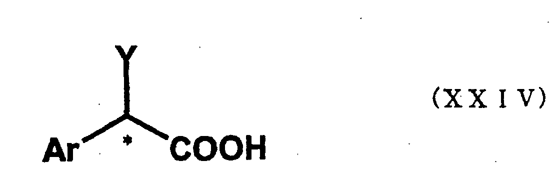 wherein Y represents any of a linear or branched alkyl group having 1 to 10 carbon atoms, a halogen atom, an alkoxy group and a hydroxyl group, Ar represents a substituted or unsubstituted phenyl group or a naphthyl group, the substituent of Ar represents any of a linear or branched alkyl group having 1 to 10 carbon atoms, a halogen atom, an alkoxy group, a hydroxyl group, a nitro group, a carboxyl group and a sulfonate group, and the symbol * represents an asymmetric carbon.

Particularly preferable examples of the optically active 2-aryl-2-substituted acetic acid represented by the general formula (XXIV) include an optically active mandelic acid derivative represented by the following general formula (XXII): 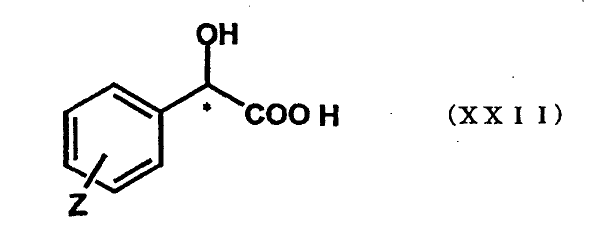 wherein Z represents any of a hydrogen atom, a linear or branched alkyl group having 1 to 10 carbon atoms, a halogen atom, an alkoxy group, a hydroxyl group, a nitro group and a benzoyl group, and the symbol * represents an asymmetric carbon.

Such an optically active mandelic acid derivative can be prepared by any process, and examples thereof include processes described in Japanese Patent Application, First Publication No. Hei 4-99496 A and Japanese Patent Application, First Publication No. Hei 4-222591 A.

The optical resolution process in this step will now be described in detail.

First, a diastereomer salt of an amino alcohol in the form of a racemate obtained in the step F with an optically active organic acid is formed in a solvent. Then, a slightly soluble diastereomer salt is deposited and a solution of an optically active amino alcohol and an optically active organic acid is obtained by solid liquid separation.

After dissolving the optically active organic acid in a suitable solvent, an equimolar to 2-fold molar amount of a racemate of amino alcohol represented by the general formula (III) is directly added dropwise or added dropwise after diluting with a suitable solvent to form a diastereomer salt of the optically active organic acid. The optically active organic acid may be added to the amino alcohol represented by the general formula (III). The mixing temperature is not specifically limited, but is preferably from 0 to 100°C, and more preferably from 10 to 80°C.

The resulting salt solution is substituted or concentrated and then cooled to form a crystal. In this case, the same kind of the diastereomer salt can also be deposited by adding a small amount of a salt crystal with high optical purity as a seed crystal. The seed crystal preferably has high optical purity and the amount is preferably from about 0.01 to 1% by weight based on the amount of the solute. Even if any seed crystal is not added, crystallization of the diastereomer salt in a supersaturated state naturally occurs to deposit the same kind of the diastereomer salt as in the case of the addition of the seed crystal. The diastereomer salt obtained by the solid liquid separation can be optionally recrystallized from a suitable solvent such as ethanol to form a diastereomer salt with higher optical purity.

After obtaining the diastereomer salt, as described above, an optically active amino alcohol can be obtained by salt dissociation using a conventionally known process.

That is, salt dissociation is conducted by contacting a solution comprising an optically active amino alcohol, which is obtained by separating the resulting diastereomer salt, an optically active organic acid and a solvent with an alkali. After cooling, solid liquid separation is conducted to recover an optically active amino alcohol from the filtrate. The optical resolution is completed in the manner described above. Furthermore, the resulting optically active organic acid alkali salt is preferably contacted with a solvent and an acid to dissolve in them, and cooled to form an optically active organic acid in the form of a crystal, which is subjected to solid liquid separation to recover an optically active organic acid.

Examples of the solvent used in the optical resolution include water; various alcohols such as methanol, ethanol, isopropanol, n-propanol and butanol; ethers such as diethyl ether, isopropylether, tetrahydrofuran and dioxane; ketones such as acetone and methyl ethyl ketone; esters such as methyl acetate and ethyl acetate; nitrogen-containing solvents such as acetonitrile and dimethylformamide; halogenated hydrocarbons such as dichloromethane, dichloroethane and chloroform; and mixtures thereof. Among these solvents, water; various alcohols such as methanol, ethanol, isopropanol, n-propanol and butanol; and mixtures thereof are preferable.

Examples of the alkali used in the salt dissociation of the diastereomer salt include alkali metal hydroxide, alkali earth metal hydroxide, alkali metal alcoholate and alkali earth metal alcoholate. Among these alkalis, alkali metal hydroxide and alkali metal alcoholate are preferable.

Examples of the acid used to recover the optically active organic acid from the optically active organic acid alkali salt include mineral acids such as hydrochloric acid, nitric acid and sulfuric acid. When water is used as the solvent, the pH is preferably 3 or lower, and more preferably from 1 to 2, after the addition of the acid

The temperature upon the solid liquid separation is preferably 40°C or lower, and more preferably from 0 to 10°C.

### (Step H)

As described above, the optically active amino alcohol in the form of the S-isomer (or optically active amino alcohol in the form of the R-isomer) represented by the general formula (VII) can be prepared from the ketone compound represented by the general formula (VI) obtained in the step E through the steps F and G, and also can be directly prepared from the ketone compound represented by the general formula (VI) through the step H.

The step H is the step of directly obtaining an optically active amino alcohol by asymmetric reduction of the ketone compound represented by the general formula (VI).

The optically active amino alcohol can be easily prepared by reacting the ketone compound represented by the general formula (VI) obtained in the step E with hydrogen in the presence of an asymmetrical hydrogenation catalyst containing transition metal, a base and an optically active nitrogen-containing compound, thereby conducting asymmetrical hydrogenation of the ketone compound.

As described above, the use of three components such as asymmetrical hydrogenation catalyst containing transition metal, base and optically active nitrogen-containing compound, asymmetrical hydrogenation reaction enables the reaction to proceed smoothly and high asymmetrical yield, and thus amino alcohol with sufficient reaction activity and high optical purity can be obtained.

Examples of the asymmetrical hydrogenation catalyst used in the step H include complexes of the group VIII transition metal. Among these, those having an optically active ligand are preferable. Specifically, those represented by the following general formula (XXV):

**M**^{**1**} **XmLn** (XXV)

wherein M¹ represents group VIII transition metal such as ruthenium, rhodium, iridium, palladium or platinum, X represents a hydrogen atom, a halogen atom, a carboxyl group, hydroxy group or an alkoxy group, L represents an optically active ligand such as optically active phosphine ligand or optically active organoarsenic compound ligand, and m and n represent an integer, are preferable.

Among these asymmetrical hydrogenation catalysts represented by the general formula (XXV), those wherein M¹ as the group VIII transition metal is ruthenium are preferable.

Specific examples of L as the optically active ligand include 2,2'-bis(diphenylphosphino)-1,1'-binaphthyl (BINAP), BINAP derivatives having the substituent such as alkyl group or aryl group on the naphthyl ring of BINAP (for example, 2,2'-bis(diphenylphosphino)-6,6'-dimethyl-1,1'-binaphthyl), BINAP derivatives wherein the naphthyl ring of BINAP is partially hydrogenated (for example, 2,2'-bis(diphenylphosphino)-5,6,7,8,5',6',7'8'-octahydro-1,1'-binaphthyl), BINAP derivatives wherein the benzene ring has 1 to 5 substituents such as alkyl group on the phosphorus atom of BINAP (for example, 2,2'-bis(di-p-tolylphosphino)-1,1'-binaphthyl), 2,2'-bis(di-3,5-xylylphosphino)-1,1'-binaphthyl, 2,2'-bis(dicyclohexylphosphino)-6,6'-dimethyl-1,1'-biphenyl,1-[1',2-bis-(diphenylphosphino)ferrocenyl]ethyldiamine, 2,3-bis(diphenylphosphino)butane, 1-cyclohexyl-1,2-bis(diphenylphosphino)ethane, 1-substituted-3,4-bis(diphenylphosphino)pyrrolidine, 2,3-0-isopropylidene-2,3-dihydroxy-1,4-bis(diphenylphosphino)butane, 1,2-bis[(o-methoxyphenyl)phenylphosphino]ethane, (substituted-1,2-bis(phospholano)benzene), 5,6-bis(diphenylphosphino)-2-norbornene, N,N'-bis-(diphenylphosphino)-N,N'-bis(1-phenylethyl)ethylenediamine, 1,2-bis(diphenylphosphino)propane and 2,4-bis(diphenylphosphino)pentane. Also PHANPHOS derivatives such as 4,12-bis(diphenylphosphino)[2.2]-paracyclophane can be used. There can also be used a monodentate optically active phosphine ligand represented by the general formula: PR¹¹R¹²R¹³ (optically active phosphine ligand wherein all of R¹¹ to R¹³ are different substituents, or optically active phosphine ligand wherein at least one of R¹¹ to R¹³ is an optically active group). Also a bidentate phosphine ligand may be used. n is preferably from 3 to 4 in the monodentate phosphine ligand, while n is preferably from 1 to 2 in the bidentate phosphine ligand.

The amount of the asymmetrical hydrogenation catalyst in the step H varies depending on the reaction vessel, reaction mode or economic efficiency, and a molar ratio of the amount of the asymmetrical hydrogenation catalyst to that of the carbonyl compound as a reactant is preferably from 1/100,000 to 1/100, and more preferably from 1/10,000 to 1/500. The molar ratio of less than the above range is not preferred because the reaction time increases, while the molar ratio of more than the above range is not preferred because the cost of the catalyst increases.

Examples of the base used in the step H include metal compounds represented by the following general formula (XXVI):

**M**^{**2**}**Y** (XXVI)

wherein M² represents alkali metal or alkali earth metal and Y represents any of a hydroxyl group, an alkoxy group, a mercapto group and a naphthyl group, and quaternary ammonium salts of alkali metal or alkali earth metal (for example, quaternary ammonium hydroxide).

Specific examples thereof include KOH, KOCH₃, KOCH(CH₃)₂, KC₁₀H₇, LiOH, LiOCH₃, LiOCH(CH₃)₂, (CH₃)₄N⁺OH⁻ and C₆H₅CH₂N (CH₃)₃⁺OH⁻.

The amount of the base is preferably from 0.5 to 100 equivalents, and more preferably from 2 to 40 equivalents, based on the asymmetrical hydrogenation catalyst. An amount of less than the above range is not preferred because the reaction time increases, while an amount of more than the above range is not preferred because the catalyst is likely to be deactivated.

Examples of the optically active nitrogen-containing compound to be used include optically active amine compounds. For example, there can be exemplified amine compounds represented by the general formula: NR¹⁴R¹⁵R¹⁶, such as optically active monoamine wherein at least one of substituents is an optically active group and the rest represent any group among a hydrogen atom, a saturated or unsaturated hydrocarbon group and an aryl group, and optically active diamine compound represented by the following general formula (XXVII): 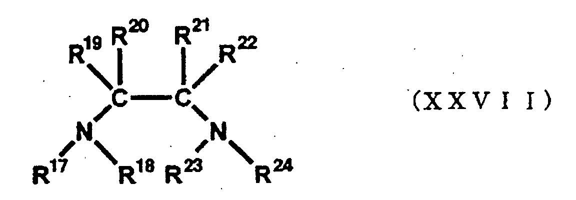 wherein R¹⁷, R¹⁸, R²³ and R²⁴ each independently represents a hydrogen atom, a saturated or unsaturated hydrocarbon group, an aryl group, an urethane group or a sulfonyl group, R¹⁹, R²⁰, R²¹ and R²² each independently represents a hydrogen atom, an alkyl group, an aromatic mono- or polycyclic group, a saturated or unsaturated hydrocarbon group or a cyclic hydrocarbon group, R¹⁹, R²⁰, R²¹ and R²² may be the same or different so that carbon, to which these substituents are attached, constitutes an asymmetric center.

Specific examples thereof include optically active diamine compounds such as optically active 1,2-diphenylethylenediamine, 1,2-cyclohexanediamine, 1,2-cycloheptanediamine, 2,3-dimethylbutanediamine, 1-methyl-2,2-diphenylethylenediamine, 1-isobutyl-2,2-diphenylethylenediamine, 1-isopropyl-2,2-diphenylethylenediamine, 1-methyl-2,2-di(p-methoxyphenyl)ethylenediamine, 1-isobutyl-2,2-di(p-methoxyphenyl)ethylenediamine, 1-isopropyl-2,2-di(p-methoxyphenyl)ethylenediamine, 1-benzyl-2,2-di(p-methoxyphenyl)ethylenediamine, 1-methyl-2,2-dinaphthylethylenediamine, 1-isobutyl-2,2-dinaphthylethylenediamine and 1-isopropyl-2,2-dinaphthylethylenediamine; and optically active diamine compounds wherein one or two substituents among substituents R¹⁷ to R²³ represents a sulfonyl group or an urethane group. As the optically active diamine, optically active propanediamine, butanediamine and phenylene diamine derivative can be used, in addition to the exemplified optically active ethylenediamine derivatives.

The amount of the optically active amine compound is preferably from 1 to 4 equivalents, and more preferably from 2 to 4 equivalents, based on the asymmetrical hydrogenation catalyst in the case of the monoamine compound. In the case of the diamine compound, the amount is preferably from 0.5 to 2.5 equivalents, and more preferably from 1 to 2 equivalents. The amount of less than the above range is not preferred because the reaction proceeds slowly, while the amount of more than the above range is not preferred because the catalyst is likely to be deactivated.

In the step H, a combination of absolute structure of the optically active ligand and absolute configuration of the optically active nitrogen-containing compound in the asymmetrical hydrogenation catalyst is important so as to achieve high asymmetrical yield. For example, (S)-3-N-benzylmethylamino-1-(2-thienyl)-1-propanol can be obtained in high yield by the combination of the R-phosphine ligand and R,R-diamine. The combination of the R-phosphine ligand and S,S-diamine enables the reaction to proceed smoothly, but causes severe decrease in asymmetrical yield.

The liquid solvent used in the step H is not specifically limited as long as it can dissolve reacting raw materials and catalysts, and examples thereof include aromatic hydrocarbon solvents such as toluene and xylene; aliphatic hydrocarbon solvents such as pentane and hexane; halogen-containing hydrocarbon solvents such as methylene chloride; ether solvents such as ether and tetrahydrofuran; alcohol solvents such as methanol, ethanol, 2-propanol, butanol and benzyl alcohol; and organic solvents containing a hetero atom such as acetonitrile, DMF and DMSO. Among these solvents, alcohol solvents are preferable because an alcohol is produced. Among these solvents, 2-propanol is particularly preferable.

The amount of the solvent to be used is decided by solubility of the reactant and economic efficiency. In the case of 2-propanol, the reaction can be conducted at a concentration of the reactant within a wide range from low concentration of 1% by weight or less to the concentration closer to no solvent, but the amount of the solvent is preferably from 20 to 50% by weight. When the amount of the solvent is less than the above range, productivity may deteriorate. On the other hand, when the amount of the solvent is more than the above range, catalytic action may reduced.

As the hydrogen source, a hydrogen gas, or a hydrogen donative organic or inorganic compound can be used.

In the step H, when the hydrogen gas is used, a hydrogen pressure is preferably from 1 to 100 kg/cm², and more preferably from 3 to 30 kg/cm², in terms of a relative pressure. When the hydrogen pressure is lower than the above range, the reaction proceeds slowly. On the other hand, when the hydrogen pressure is higher than the above range, economic efficiency is reduced, which is not preferred. It is possible to maintain high activity even at low hydrogen pressure of 10 kg/cm² or lower.

Taking into account economic efficiency, the reaction temperature is preferably from -30 to 100°C, and the reaction can be carried out even at room temperature within a range from about 10 to 40°C. The reaction time varies depending on reaction conditions such as reactant concentration, temperature and pressure, and the reaction is completed within several minutes to 10 hours.

The reaction in the step H can also be carried out in a batch-wise or continuous manner.

The hydrogen donor organic or inorganic compound means a compound capable of donating hydrogen by means of a thermal action or a catalytic action. Such a hydrogen donor compound is not specifically limited and preferred examples thereof include alcohol compounds such as methanol, ethanol, 1-propanol, 2-propanol, butanol and benzyl alcohol; formic acid and salts thereof, for example, combination of amines; unsaturated hydrocarbon or heterocyclic compounds having partially saturated hydrocarbon, such as tetralin and decalin; hyroquinone and phosphorous acid. Among these compounds, alcohol compounds are preferable and 2-propanol is more preferable.

The amount of the organic compound as the hydrogen source is preferably 0.1% by weight or more in view of solubility of the reactant and is preferably 30% by weight or less in view of economic efficiency, although the reaction can be conducted at the reactant concentration within a range from 0.01% to 100% by weight. When formic acid or a combination of formic acid and an amine is used as the hydrogen source, the solvent may be used or not. When the solvent is used, aromatic hydrocarbon solvents such as toluene and xylene; and organic solvents containing hetero atom, such as acetonitrile, DMF and DMSO can be used.

3-N-methylamino-1-(2-thienyl)-1-propanol in the form of a racemate or an optically active substance can be easily prepared from dialkylamino alcohol (propanolamine derivative represented by the general formula (III) or (VII)), which is a propanolamine derivative in the form of the racemate or optically active substance thus obtained, via various propanolamine derivatives through the dealkylation step shown in the scheme 2.

### (Step J)

The step J is the step of preparing a propanolamine derivative in the form of a racemate or an optically active substance represented by the following general formula (XI): 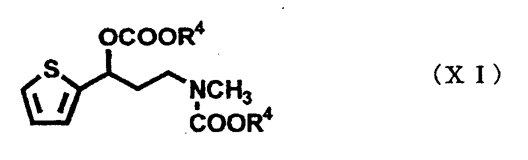 by reacting a propanolamine derivative represented by the general formula (III) with a chloroformic acid derivative represented by the following general formula (IX):

**CICOOR**^{**4**} (IX)

in the presence of a base. In the general formulas (IX) and (XI), R⁴ represents an alkyl group having 1 to 8 carbon atoms which may have a substituent or a phenyl group which may have a substituent.

Specific examples the chloroformic acid derivative used in the step J include ethyl chloroformate, methyl chloroformate, phenyl chloroformate, 2,2,2-trichloroethyl chloroformate, 2-chloroethyl chloroformate, 2-iodoethyl chloroformate, butyl chloroformate, propyl chloroformate, benzyl chloroformate, nitrobenzyl chloroformate and 2,2-dichloroethyl chloroformate. Among these chloroformic acid derivatives, phenyl chloroformate, isopropyl chloroformate and isobutyl chloroformate are suited for the dealkylation reaction, and isopropyl chloroformate and isobutyl chloroformate are particularly preferable because a deleterious substance such as phenol is not produced as a by-product.

The amount of the chloroformic acid derivative is preferably from 1 to 6 mols, and more preferably from 1.5 to 4 mols, per mol of the amino alcohol derivative represented by the general formula (III).

Examples of the base include hydroxides of amines or alkali metal or their salts with weak acid; hydroxides of alkali earth metal or their salts with weak acid; and hydroxides of quaternary ammonium or their salts with weak acid. Among these bases, preferable bases are ammonia; trialkylamines such as triethylamine and trimethylamine; dialkylamines such as dimethylamine and benzylmethylamine; monoalkylamines such as monomethylamine; cyclic amines such as morpholine, N-methylmorpholine and piperidine; N,N-dimethylaniline, pyridine, ammonia, 1,8-bis(dimethylamino)naphthalene, sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 0 to 5 mols, and more preferably from 0.2 to 3 mols, per mol of the chloroformic acid derivative.

The solvent is not specifically limited as long as it does not exert an adverse influence on the reaction, and examples thereof include amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; nitriles such as acetonitrile; and ethers such as butylmethylether, diisopropyl ether, tetrahydrofuran, 1,4-dioxane, 1,2-dimethoxyethane and anisole. These solvents can be used alone or in combination thereof. The amount of the solvent is industrially preferably from about 50 to 3000 parts by weight based on 100 parts by weight of the chloroformic acid derivative in view of operability and economic efficiency.

The reaction temperature varies depending on the kind of the solvent, the amount of the solvent and the chloroformic acid derivative and, as the temperature becomes higher, an increase in the amount of by-product due to the side reaction occurs. Therefore, the reaction temperature is preferably 100°C or lower, and more preferably 50°C or lower. If the temperature is too low, deterioration of operability may be caused by solidification of the reaction solution. Therefore, the reaction temperature is preferably -30°C or higher, and more preferably 0°C or higher. By controlling the these conditions, selectivity and reactivity of the dealkylation step are remarkably improved, and thus the objective compound can be obtained in very high yield and at high purity. In view of the yield, purity and amount to be used, phenyl chloroformate, isopropyl chloroformate and isobutyl chloroformate are preferable, and isopropyl chloroformate and isobutyl chloroformate are particularly preferable since a deleterious substance such as phenol is not produced as a by-product.

### (Step K)

The step K is the step of preparing a propanolamine derivative represented by the general formula (XV): 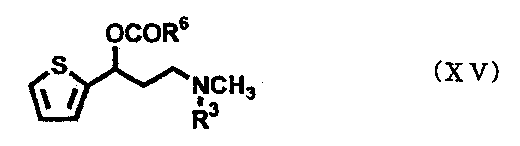 by reacting a propanolamine derivative represented by the general formula (III) with an alkylcarboxylic acid chloride or alkylcarboxylic anhydride represented by the following general formula (XIII):

**CICOR**^{**6**} (XIII)

or the general formula (XIV):

**(R**^{**6**}**CO)**_{**2**}**O** (XIV)

in the presence of a base. In the general formulas (XIII), (XIV) and (XV), R⁶ represents an alkyl group having 1 to 8 carbon atoms or phenyl group.

The acid chloride or acid anhydride serves as an acylating agent.

Specific examples of the acid chloride include acetyl chloride, propionic acid chloride, butyric acid chloride, benzoyl chloride. Among these acid chlorides, acetyl chloride is preferable. Specific examples of the acid anhydride include acetic anhydride, butyric anhydride and propionic anhydride. Among these acid anhydrides, acetic anhydride is preferable. The amount of the acid chloride or acid anhydride is preferably from 0.3 to 6 mols, and more preferably from 0.5 to 4 mols, per mol of the compound represented by the general formula (III).

Examples of the base include hydroxides of amines or alkali metal or their salts with weak acid; hydroxides of alkali earth metal or their salts with weak acid; and hydroxides of quaternary ammonium or their salts with weak acid. Among these bases, preferable bases are ammonia; trialkylamines such as triethylamine and trimethylamine; cyclic amines such as N-methylmorpholine and N,N'-dimethylpiperidine; N,N-dimethylaniline, pyridine, 1,8-bis(dimethylamino)naphthalene, sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 0 to 5 mols, and more preferably from 0.2 to 3 mols, per mol of the acylating agent.

The solvent is not specifically limited as long as it does not exert an adverse influence on the reaction, and examples thereof include amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; nitriles such as acetonitrile; ethers such as butylmethylether, diisopropyl ether, tetrahydrofuran, 1,4-dioxane, 1,2-dimethoxyethane and anisole; and water. These solvents can be used alone or in combination. The amount is not specifically limited and is preferably from about 50 to 3000 parts by weight based on 100 parts by weight of the propanolamine derivative represented by the general formula (III) in view of operability and economic efficiency.

The reaction temperature is preferably from -30 to 150°C, and more preferably from 0 to 100°C.

### (Step L)

The step L is the step of preparing a propanolamine derivative in the form of a racemate or an optically active substance, which is represented by the general formula (XVI): 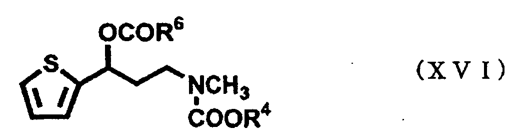 by reacting the propanolamine derivative represented by the general formula (XV) obtained in the step K with a chloroformic acid derivative represented by the following general formula (IX):

**CICOOR**^{**4**} (IX)

in the presence of a base. In the general formulas (IX) and (XVI), R⁴ represents an alkyl group having 1 to 8 carbon atoms which may have a substituent or a phenyl group which may have a substituent, and R⁶ represents an alkyl group having 1 to 8 carbon atoms or a phenyl group.

Specific examples of the chloroformic acid derivative used in the step L include ethyl chloroformate, methyl chloroformate, phenyl chloroformate, butyl chloroformate, propyl chloroformate, benzyl chloroformate, nitrobenzyl chloroformate, 2,2,2-trichloroethyl chloroformate, 2-chloroethyl chloroformate, 2,2-dichloroethyl chloroformate, 1,1-dimethyl-2,2,2-trichloroethyl chloroformate, 1,1-dimethyl-2-chloroethyl chloroformate and 1,1-dimethyl-2-bromoethyl chloroformate. Among these chloroformic acid derivatives, phenyl chloroformate, isopropyl chloroformate and isobutyl chloroformate are preferable. The amount of the chloroformic acid derivative is preferably from 0.5 to 5 mols, and more preferably from 0.7 to 4 mols, per mol of the propanolamine derivative represented by the general formula (XV).

As the base, hydroxides of amines or alkali metal or their salts with weak acid; hydroxides of alkali earth metal or their salts with weak acid; and hydroxides of quaternary ammonium or their salts with weak acid are used. Among these bases, preferable bases are ammonia,; rialkylamines such as triethylamine and trimethylamine; cyclic amines such as N-methylmorpholine and piperidine; N,N-dimethylaniline, pyridine, 1,8-bis(dimethylamino)naphthalene, sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 0 to 5 mols, and more preferably from 0.2 to 3 mols, per mol of the chloroformic acid derivative.

The solvent is not specifically limited as long as it does not exert an adverse influence on the reaction, and examples thereof include amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; nitriles such as acetonitrile; and ethers such as butylmethylether, diisopropyl ether, tetrahydrofuran, 1,4-dioxane, 1,2-dimethoxyethane and anisole. These solvents can be used alone or in combination. The amount of the solvent is industrially preferably from about 50 to 3000 parts by weight based on 100 parts by weight of the chloroformic acid derivative in view of operability and economic efficiency.

The reaction temperature is preferably from -30 to 150°C, and more preferably from 0 to 100°C.

### (Step M)

The step M is the step of preparing a propanolamine derivative represented by the general formula (XVII): 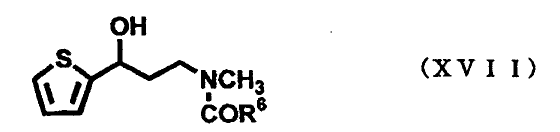 wherein R⁶ represents an alkyl group having 1 to 8 carbon atoms or a phenyl group, by reducing the propanolamine derivative represented by the general formula (XV) obtained in the step K.

The de(non)substitution benzylation reaction of the step M can be conducted by hydrogen reduction or hydrogen transfer reaction using a catalyst, reductive reaction using metallic sodium/liquid ammonia, or reductive reaction using DDQ. Among these reactions, the hydrogen reduction or hydrogen transfer reaction using a catalyst is preferably employed in view of economic efficiency and operability.

The reducing catalyst preferably contains at least one element selected from nickel, cobalt, rhodium and platinum and, for example, metal or salts such as nitrate, sulfate and chloride, and compounds such as oxide and hydroxide are preferable. These catalysts are preferable because less adverse influence of a catalyst poison action of sulfur is exerted.

Specific examples of preferable catalyst include platinum oxide, Raney nickel, rhodium, platinum, platinum-carbon, rhodium-platinum oxide and cobalt-silica gel. The amount of the catalyst is preferably from 1 to 1/1000 parts by weight, and more preferably from 1/2 to 1/100 parts by weight, based on 100 parts by weight of the O-acyl compound represented by the general formula (XV) obtained in the step K. The catalyst can be used in the form of being supported on a carrier. As the carrier, activated carbon, silica, alumina and silica alumina or the like are used and the amount of the catalyst to be supported is preferably from 5 to 70% by weight.

The reaction of the step M can be conducted in the presence of hydrogen or a hydrogen source such as formic acid or ammonium formate. In the reaction, it is preferred to use solvents which are inert to the reaction, for example, aliphatic alcohols such as methanol, ethanol, isopropanol and ethylene glycol; aliphatic ethers such as dimethoxyethane, dioxane and tetrahydrofuran; aliphatic esters such as methyl acetate, ethyl acetate and ethyl propionate; and aromatic hydrocarbons such as toluene and xylene so as to dissolve the O-acyl compound obtained in the step K. Among these solvents, aliphatic alcohols and aliphatic esters are preferable, and methanol, isopropanol and dioxane are particularly preferable in view of economic efficiency.

When the hydrogen source such as formic acid is used, the reaction can be conducted under normal pressure. When using hydrogen, a hydrogen pressure is preferably from 1 to 200 kg/cm², and more preferably from 10 to 100 kg/cm², in terms of a relative pressure.

The reaction temperature is preferably within a range from 0 to 150°C, and more preferably from 40 to 100°C. The reaction time varies depending on reaction conditions such as catalyst amount, reaction temperature and hydrogen pressure, and is preferably within 30 hours, and more preferably from 0.5 to 20 hours.

### (Step N)

The step N is the step of preparing a propanolamine derivative represented by the general formula (XVII) by conducting the cleavage reaction of the urethane moiety and the transfer reaction of the acyl group of a propanolamine derivative represented by the following general formula (XIX) : 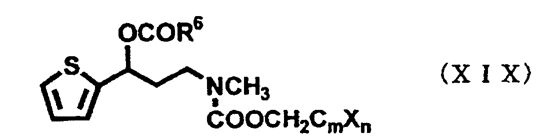 which is obtained by reacting a propanolamine derivative represented by the general formula (XV) obtained in the step K, among compounds obtained by the step L, with a chloroformic acid derivative represented by the following general formula (X).

**CICOOCH**_{**2**}**CH**_{**m**}**X**_{**n**} (X)

In the general formulas (X) and (XIX), R⁶ represents an alkyl group having 1 to 8 carbon atoms or a phenyl group, X represents a halogen atom, m and n each independently represents an integer of 0 to 3 and the sum of them is 3.

Examples of the cleaving agent used in the cleavage of urethane include zinc.

The solvent used in the step N is not specifically limited as long as it does not exert an adverse influence on the reaction, and examples thereof include amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; nitriles such as acetonitrile; and ethers such as butylmethylether, diisopropyl ether, tetrahydrofuran, 1,4-dioxane, 1,2-dimethoxyethane and anisole. These solvents can be used alone or in combination. The amount of the solvent is industrially preferably from about 50 to 3000 parts by weight based on 100 parts by weight of the propanolamine derivative represented by the general formula (XIX) in view of operability and economic efficiency.

The pH in the reaction system is preferably from 1 to 9, and more preferably from 2 to 8. The reaction temperature is preferably from -30 to 150°C, and more preferably from 0 to 100°C.

### (Step O)

The step O is the step of preparing a propanolamine derivative represented by the general formula (XII): 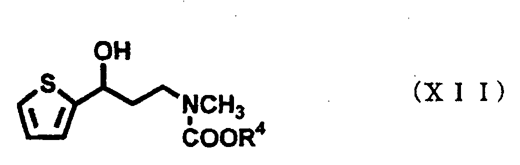 wherein R⁴ represents an alkyl group having 1 to 8 carbon atoms which may have a substituent or a phenyl group which may have a substituent, by treating the propanolamine derivative represented by the general formula (XI) obtained in the step J with a base.

Examples of the base used in the step O include hydroxides of alkali metal or their salts with weak acid; hydroxides of alkali earth metal or their salts with weak acid; and hydroxides of quaternary ammonium or their salts with weak acid. Specific examples thereof include sodium hydroxide, potassium hydroxide, sodium carbonate, sodium hydrogencarbonate, potassium carbonate, potassium hydrogencarbonate, cesium carbonate, tripotassium phosphate, tripotassium phosphate dihydrate. The amount of the base is preferably from 1 to 10 mols, and more preferably from 2 to 6 mols, per mol of the compound represented by the general formula (XI) obtained in the step J.

The solvent is not specifically limited as long as it does not exert an adverse influence on the reaction, and examples thereof include amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methyl ether, diisopropyl ether, tetrahydrofuran, 1,4-dioxane, 1,2-dimethoxyethane and anisole; and water. These solvents can be used alone or in combination. The amount of the solvent is industrially preferably from about 50 to 3000 parts by weight based on 100 parts by weight of the N,O-bis(alkoxycarbonyl) compound represented by the general formula (XI) in view of operability and economic efficiency. When using the solvent other than water, a suitable amount of water is preferably used. The amount of water is preferably from 1 to 10000 mols, and more preferably from 2 to 6000 mols, per mol of the N,O-bis(alkoxycarbonyl) compound represented by the general formula (XI). The reaction temperature is preferably from -30 to 100°C, and more preferably from 0 to 90°C.

### (Step of P)

The step P is the step of preparing a propanolamine derivative represented by the general formula (XVIII): 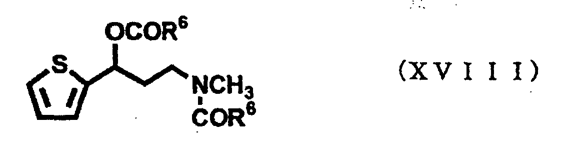 by reacting the propanolamine derivative represented by the general formula (XVII) in the step M or N with an alkylcarboxylic acid chloride or alkylcarboxylic anhydride represented by the following general formula (XIII):

**CICOR**^{**6**} (XIII)

or the general formula (XIV):

**(R**^{**6**}**CO)**_{**2**}**O** (XIV)

in the presence of a base. The acid chloride or acid anhydride serves as an acylating agent.

Examples of the acid chloride include acetyl chloride, propionic acid chloride, butyric acid chloride and benzoyl chloride. Among these acid chlorides, acetyl chloride is preferable. Examples of the acid anhydride include acetic anhydride, butyric anhydride and propionic anhydride. Among these acid anhydrides, acetic anhydride is preferable. The amount of the acid chloride or acid anhydride is preferably from 0.3 to 6 mols, and more preferably from 0.5 to 4 mols, per mol of the compound represented by the general formula (XVII).

Examples of the base include amines; hydroxides of alkali metal or their salts with weak acid; hydroxides of alkali earth metal or their salts with weak acid; and hydroxides of quaternary ammonium or their salts with weak acid. Specific examples thereof include trialkylamines such as ammonia, triethylamine and trimethylamine; cyclic amines such as N-methylmorpholine and N,N'-dimethylpiperidine; N,N-dimethylaniline, pyridine, 1,8-bis(dimethylamino)naphthalene, sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 0 to 5 mols, and more preferably from 0.2 to 3 mols, per mol of the chloroformic acid derivative.

The solvent is not specifically limited as long as it does not exert an adverse influence on the reaction, and examples thereof include amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; nitriles such as acetonitrile; ethers such as butyl methyl ether, diisopropyl ether, tetrahydrofuran, 1,4-dioxane, 1,2-dimethoxyethane and anisole; and water. These solvents can be used alone or in combination. The amount of the solvent is not specifically limited, and is industrially preferably from about 50 to 3000 parts by weight based on 100 parts by weight of the propanolamine derivative represented by the general formula (XVII) in view of operability and economic efficiency.

The reaction temperature is preferably from -30 to 150°C, and more preferably from 0 to 100°C.

### (Step Q)

The step Q is the step of preparing 3-N-methylamino-1-(2-thienyl)-1-propanol represented by the general formula (IV) by hydrolyzing the propanolamine derivative represented by the general formula (XII) obtained in the step O in the presence of a base. The hydrolysis reaction is easily handled because heavy metal such as Zn is not required.

Examples of the base used in the step Q include hydroxides of alkali metal or their salts with weak acid; hydroxides of alkali earth metal or their salts with weak acid; and hydroxides of quaternary ammonium or their salts weak acid. Specific examples thereof include sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 1 to 10 mols, and more preferably from 2 to 6 mols, per mol of the N-alkoxycarbonylalcohol compound represented by the general formula (XII) obtained in the step O.

The solvent is not specifically limited as long as it does not exert an adverse influence on the reaction, and examples thereof include alcohols such as methanol, ethanol, propanol, isopropanol and butanol; amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methyl ether, diisopropyl ether, tetrahydrofuran, 1,4-dioxane, 1,2-dimethoxyethane and anisole; and water. These solvents can be used alone or in combination. The amount of the solvent is industrially preferably from about 50 to 3000 parts by weight based on 100 parts by weight of the N-alkoxycarbonyl alcohol compound represented by the general formula (XII) in view of operability and economic efficiency. When using a solvent other than water, a suitable amount of water can be added thereto. The amount of water is preferably from 1 to 10000 mols, and more preferably from 2 to 6000 mols, per mol of the N-alkoxycarbonyl alcohol compound represented by the general formula (XII). The reaction temperature is preferably from 0 to 200°C, and more preferably from 10 to 120°C.

### (Step R)

The step R is the step of preparing 3-N-methylamino-1-(2-thienyl)-1-propanol represented by the general formula (IV) by hydrolyzing the propanolamine derivative represented by the general formula (XI) obtained in the step J in the presence of a base. By the hydrolysis in the presence of the base, a hydroxyl group and an urethane group of the propanolamine derivative represented by the general formula (XI) can be simultaneously deprotected to prepare 3-N-methylamino-1-(2-thienyl)-1-propanol represented by the general formula (IV). The hydrolysis reaction is easily handled because heavy metal such as Zn is not required.

Examples of the base used in the step R include hydroxides of alkali metal or their salts with weak acid; hydroxides of alkali earth metal or their salts with weak acid; and hydroxides of quaternary ammonium or their salts with weak acid. Specific examples thereof include sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 1 to 10 mols, and more preferably from 2 to 6 mols, per mol of the N,O-bis(alkoxycarbonyl) compound represented by the general formula (XI).

The solvent is not specifically limited as long as it does not exert an adverse influence on the reaction, and examples thereof include alcohols such as methanol, ethanol, propanol, isopropanol and butanol; amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methyl ether, diisopropyl ether, tetrahydrofuran, 1,4-dioxane, 1,2-dimethoxyethane and anisole; and water. These solvents can be used alone or in combination. The amount of the solvent is industrially preferably from about 50 to 3000 parts by weight based on 100 parts by weight of the N,O-bis(alkoxycarbonyl) compound represented by the general formula (XI) in view of operability and economic efficiency. When using the solvent other than water, a suitable amount of water can also be added thereto. The amount of water is preferably from 0 to 10000 mols, and more preferably from 2 to 6000 mols, per mol of the N,O-bis(alkoxycarbonyl) compound represented by the general formula (XI).

The reaction temperature is preferably from 0 to 200°C, and more preferably from 10 to 150°C.

### (Step S)

The step S can be roughly classified into three steps, for example, steps S-1, S-2 and S-3, and these steps will now be described, respectively.

### <Step S-1>

The step S-1 is the step of preparing 3-N-methylamino-1-(2-thienyl)-1-propanol represented by the general formula (IV) by reductively eliminating the substituent R³ of a propanolamine derivative represented by the general formula (III).

The de(non)substitution benzylation reaction of this step can be conducted by a hydrogen reduction or hydrogen transfer reaction using a catalyst, a reductive reaction using metallic sodium/liquid ammonia or a reductive reaction using DDQ. Among these reactions, the hydrogen reduction or hydrogen transfer reaction using the catalyst is preferably employed in view of economic efficiency and operability.

The reducing catalyst preferably contains at least one element selected from nickel, cobalt, rhodium and platinum and, for example, metal or salts such as nitrate, sulfate and chloride, and compounds such as oxide and hydroxide are preferable. These catalysts are preferable because less adverse influence of a catalyst poison action of sulfur is exerted.

Specific examples of preferable catalyst include platinum oxide, Raney nickel, rhodium, platinum, platinum-carbon, rhodium-platinum oxide and cobalt-silica gel. The amount of the catalyst is preferably from 10 to 1/1000 parts by weight, and more preferably from 5 to 1/100 parts by weight, based on 100 parts by weight of the compound represented by the general formula (III). The catalyst can be used in the form of being supported on a carrier. As the carrier, activated carbon, silica, alumina and silica alumina are used and the amount of the catalyst to be supported is preferably from 5 to 70% by weight.

The reaction can be conducted in the presence of hydrogen or a hydrogen source such as formic acid or ammonium formate. In the reaction, it is preferred to use solvents which are inert to the reaction, for example, aliphatic alcohols such as methanol, ethanol, isopropanol and ethylene glycol; aliphatic ethers such as dimethoxyethane, dioxane and tetrahydrofuran; and aliphatic esters such as methyl acetate, ethyl acetate and ethyl propionate; and aromatic hydrocarbons such as toluene and xylene so as to dissolve the compound represented by the general formula (III). Among these solvents, aliphatic alcohols and aliphatic esters are preferable, and methanol, isopropanol and dioxane are particularly preferable in view of economic efficiency.

When the hydrogen source such as formic acid is used, the reaction can be conducted under normal pressure. When using hydrogen, a hydrogen pressure is preferably from 1 to 200 kg/cm², and more preferably from 10 to 100 kg/cm², in terms of a relative pressure. The reaction temperature is preferably within a range from 0 to 180°C, and more preferably from 40 to 150°C. The reaction time varies depending on reaction conditions such as catalyst amount, reaction temperature and hydrogen pressure, and is preferably within 500 hours, and more preferably from 0.5 to 400 hours.

### <Step S-2>

The step S-2 is the step of preparing 3-N-methylamino-1-(2-thienyl)-1-propanol represented by the general formula (IV) by reacting a propanolamine derivative represented by the general formula (III) with a chloroformic acid derivative represented by the following general formula (IX):

**CICOOR**^{**4**} (IX)

wherein R⁴ represents an alkyl group having 1 to 8 carbon atoms which may have a substituent or a phenyl group which may have a substituent, in the presence of a base, and hydrolyzing the intermediate without isolation. In this case, the intermediate can be subjected to the following hydrolysis step without protecting a hydroxyl group of the propanolamine derivative represented by the general formula (III) with another protective group and 3-N-methylamino-1-(2-thienyl)-1-propanol can be easily prepared, and therefore this is superior step.

Specific examples of the chloroformic acid derivative used in this step include ethyl chloroformate, methyl chloroformate, phenyl chloroformate, 2,2,2-trichloroethyl chloroformate, 2-chloroethyl chloroformate, 2-iodoethyl chloroformate, butyl chloroformate, propyl chloroformate, benzyl chloroformate, nitrobenzyl chloroformate, 2,2,2-trichloroethyl chloroformate, 2-chloroethyl chloroformate, 2,2-dichloroethyl chloroformate, 1,1-dimethyl-2,2,2-trichloroethyl chloroformate, 1,1-dimethyl-2-chloroethyl chloroformate and 1,1-dimethyl-2-bromoethyl chloroformate. Among these chloroformic acid derivatives, phenylchloroformate, iso-propylchloroformate and isobutylchloroformate are preferable. The amount of the chloroformic acid derivative is preferably from 1 to 6 mols, and more preferably from 1.5 to 4 mols, per mol of the amino alcohol derivative represented by the general formula (III).

Examples of the base used in the step of reacting a propanolamine derivative represented by the general formula (III) with a chloroformic acid derivative include amines; hydroxides of alkali metal or their salts with weak acid; hydroxides of alkali earth metal or their salts with weak acid; and hydroxides of quaternary ammonium or their salts with weak acid. Among these bases, preferable bases are ammonia; trialkylamines such as triethylamine and trimethylamine; dialkylamines such as dimethylamine and benzylmethylamine; monoalkylamines such as monomethylamine; cyclic amines such as morpholine, N-methylmorpholine and piperidine; N,N-dimethylaniline, pyridine, ammonia, 1,8-bis(dimethylamino)naphthalene, sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 0 to 5 mols, and more preferably from 0.2 to 3 mols, per mol of the chloroformic acid derivative.

The solvent used in the step of reacting a propanolamine derivative represented by the general formula (III) with a chloroformic acid derivative is not specifically limited as long as it does not exert an adverse influence on the reaction, and examples thereof include amides such as dimethylformamide and dimethylacetamide; pyrrolidones such as N-methyl-2-pyrrolidone; ketone· sulfoxides such as acetone, ethyl methyl ketone and dimethyl sulfoxide; aromatic hydrocarbons such as benzene, toluene, xylene and mesitylene; nitriles such as acetonitrile; and ethers such as t-butyl methyl ether, diisopropyl ether, tetrahydrofuran, 1,4-dioxane, 1,2-dimethoxyethane and anisole. When the replacement of the solvent is not conducted in the following hydrolysis step, aromatic hydrocarbons such as benzene, toluene, xylene and mesitylene; and ethers such as t-butyl methyl ether, diisopropyl ether, tetrahydrofuran, 1,4-dioxane, 1,2-dimethoxyethane and anisole are preferable. These solvents can be used alone or in combination. The amount of the solvent is industrially preferably from about 50 to 3000 parts by weight based on 100 parts by weight of the chloroformic acid derivative in view of operability and economic efficiency.

The reaction temperature in the step of reacting a propanolamine derivative represented by the general formula (III) with a chloroformic acid derivative varies depending on the kind of the solvent, the amount of the solvent and the chloroformic acid derivative and, as the temperature becomes higher, an increase in the amount of by-product due to the side reaction occurs. Therefore, the reaction temperature is preferably 100°C or lower, and more preferably 50°C or lower. If the temperature is too low, deterioration of operability may be caused by solidification of the reaction solution. Therefore, the reaction temperature is preferably -30°C or higher, and more preferably 0°C or higher. By controlling these conditions, selectivity and reactivity of the dealkylation step are remarkably improved, and thus the objective compound can be obtained in very high yield and at high purity. In view of the yield, purity and amount to be used, phenyl chloroformate, isopropyl chloroformate and isobutyl chloroformate are preferable, and isopropyl chloroformate and isobutyl chloroformate are particularly preferable since a deleterious substance such as phenol is not produced as by-product.

Examples of the base used in the hydrolysis step include hydroxides of alkali metal or their salts with weak acid; hydroxides of alkali earth metal or their salts with weak acid; and hydroxides of quaternary ammonium or their salts with weak acid. Specific examples thereof include sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. Among these bases, hydroxides of alkali metal such as sodium hydroxide and potassium hydroxide are preferable. The amount of the base is preferably from 1 to 10 mols, and more preferably from 2 to 6 mols, per mol of the chloroformic acid derivative.

The solvent used in the hydrolysis step is not specifically limited as long as it does not exert an adverse influence on the reaction, and examples thereof include alcohols such as methanol, ethanol, propanol, isopropanol and butanol; amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methyl ether, diisopropyl ether, tetrahydrofuran, 1,4-dioxane, 1,2-dimethoxyethane and anisole; and water. These solvents can be used alone or in combination. The amount of the solvent is industrially preferably from about 50 to 3000 parts by weight based on 100 parts by weight of the compound represented by the general formula (III) in view of operability and economic efficiency. When using the solvent other than water, a suitable amount of water can also be added thereto. The amount of water is preferably from 0 to 10000 mols, and more preferably from 0 to 6000 mols, per mol of the compound represented by the general formula (III).

The reaction temperature of the hydrolysis step is preferably from 0 to 200°C, and more preferably from 10 to 150°C.

### <Step S-3>

The step S-3 is the step of preparing 3-N-methylamino-1-(2-thienyl)-1-propanol represented by the general formula (IV) by reacting a propanolamine derivative represented by the general formula (III) with a chloroformic acid derivative represented by the following general formula (X):

**CICOOCH**_{**2**}**CH**_{**m**}**X**_{**n**} (X)

wherein X represents a halogen atom, m and n each independently represents an integer of 0 to 3 and the sum of them is 3, in the presence of a base, and subjecting the intermediate to dehalogenation/alkyloxycarbonylation and hydrolysis without isolation.

Specific examples of the chloroformic acid derivative used in this step include 2,2,2-trichloroethyl chloroformate, 2-chloroethyl chloroformate, 2,2-dichloroethyl chloroformate, 1,1-dimethyl-2,2,2-trichloroethyl chloroformate, 1,1-dimethyl-2-chloroethyl chloroformate and 1,1-dimethyl-2-bromoethyl chloroformate. Among these chloroformic acid derivatives, 2,2,2-trichloroethyl chloroformate is preferable. The amount of the chloroformic acid derivative is preferably from 0.5 to 5 mols, and more preferably from 0.7 to 4 mols, per mol of the amino alcohol derivative represented by the general formula (III).

Examples of the base used in the step of reacting a propanolamine derivative represented by the general formula (III) with a chloroformic acid derivative include amines; hydroxides of alkali metal or their salts with weak acid; hydroxides of alkali earth metal or their salts with weak acid; and hydroxides of quaternary ammonium or their salts with weak acid. Among these bases, preferable bases are ammonia; trialkylamines such as triethylamine and trimethylamine; cyclic amines such as N-methylmorpholine and piperidine; N,N-dimethylaniline, pyridine, 1,8-bis(dimethylamino)naphthalene, sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 0 to 5 mols, and more preferably from 0.2 to 3 mols, per mol of the chloroformic acid derivative.

The solvent used in the step of reacting a propanolamine derivative represented by the general formula (III) with a chloroformic acid derivative is not specifically limited as long as it does not exert an adverse influence on the reaction, and examples thereof include amides such as dimethylformamide and dimethylacetamide; pyrrolidones such as N-methyl-2-pyrrolidone; ketone sulfoxides such as acetone, ethyl methyl ketone and dimethyl sulfoxide; aromatic hydrocarbons such as benzene, toluene, xylene and mesitylene; nitriles such as acetonitrile; and ethers such as butyl methyl ether, diisopropyl ether, tetrahydrofuran, 1,4-dioxane, 1,2-dimethoxyethane and anisole. These solvents can be used alone or in combination. The amount of the solvent is industrially preferably from about 50 to 3000 parts by weight based on 100 parts by weight of the chloroformic acid derivative in view of operability and economic efficiency.

The reaction temperature in the step of reacting a propanolamine derivative represented by the general formula (III) with a chloroformic acid derivative is preferably from -30 to 150°C, and more preferably from 0 to 100°C.

In the propanolamine derivative obtained in the step of reacting a propanolamine derivative represented by the general formula (III) with a chloroformic acid derivative, the dealkoxycarbonylation of the carbamate moiety can be easily conducted reductively without isolation. Simultaneously, transfer of an acyl group can be conducted, and thus the propanolamine derivative can be easily converted into an N-acyl compound in the form of a racemate or an optically active substance.

Examples of the dealkoxycarbonylating agent of the carbamate moiety include zinc/acetic acid, sodium/ammonia, boron trifluoride etherate/trifluoroacetic acid and alcohol. Among these dealkoxycarbonylating agents, zinc/acetic acid is preferable.

Examples of the solvent used in the dealkoxycarbonylation step include solvents exemplified in the step of reacting a propanolamine derivative represented by the general formula (III) with a chloroformic acid derivative.

The pH in the reaction system in the dealkoxycarbonylation step is preferably from 1 to 9, and more preferably from 2 to 8. The reaction temperature is preferably from -30 to 150°C, and more preferably from 0 to 100°C.

### (Step T)

The step T is the step of preparing 3-N-methylamino-1-(2-thienyl)-1-propanol in the form of a racemate or an optically active substance by hydrolyzing O-acyl N-alkoxycarbonyl compound in the form of a racemate or an optically active substance, which is represented by general formula (XVI), obtained in the step L in the presence of a base.

Examples of the base used in the step T include hydroxide of alkali metal or their salts with weak acid; hydroxides of alkali earth metal or their salts with weak acid; and hydroxides of quaternary ammonium or their salts with weak acid. Specific examples thereof include sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 1 to 10 mols, and more preferably from 2 to 6 mols, per mol of the O-acyl N-alkoxycarbonyl compound represented by the general formula (XVI).

The solvent is not specifically limited as long as it does not exert an adverse influence on the reaction, and examples thereof include alcohols such as methanol, ethanol, propanol, isopropanol and butanol; amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methylether, diisopropyl ether, tetrahydrofuran, 1,4-dioxane, 1,2-dimethoxyethane and anisole; and water. These solvents can be used alone or in combination. The amount of the solvent is industrially preferably from about 50 to 3000 parts by weight based on 100 parts by weight of the O-acyl N-alkoxycarbonyl compound represented by the general formula (XVI) in view of operability and economic efficiency. When using the solvent other than water, a suitable amount of water can also be added thereto. The amount of water is preferably from 1 to 10000 mols, and more preferably from 2 to 6000 mols, per mol of the O-acyl N-alkoxycarbonyl compound represented by the general formula (XVI).

The reaction temperature is preferably from 0 to 200°C, and more preferably from 10 to 150°C.

### (Step U)

The step U is the step of preparing 3-N-methylamino-1-(2-thienyl)-1-propanol represented by the general formula (IV) by hydrolyzing the propanolamine derivative represented by the general formula (XVIII) obtained in the step P in the presence of a base.

Examples of the base used in the step U include hydroxides of alkali metal or their salts with weak acid; hydroxides of alkali earth metal or their salts with weak acid; and hydroxides of quaternary ammonium or their salts with weak acid. Specific examples thereof include sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 1 to 10 mols, and more preferably from 2 to 6 mols, per mol of the N,O-diacyl compound represented by the general formula (XVIII).

The solvent is not specifically limited as long as it does not exert an adverse influence on the reaction, and examples thereof include alcohols such as methanol, ethanol, propanol, isopropanol and butanol; amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methyl ether, diisopropyl ether, tetrahydrofuran, 1,4-dioxane, 1,2-dimethoxyethane and anisole; and water. These solvents can be used alone or in combination. The amount of the solvent is industrially preferably from about 50 to 3000 parts by weight based on 100 parts by weight of the N,O-diacyl compound represented by the general formula (XVIII) in view of operability and economic efficiency. When using the solvent other than water, a suitable amount of water can also be added thereto. The amount of water is preferably from 0 to 10000 mols, and more preferably from 2 to 6000 mols, per mol of the N,O-diacyl compound represented by the general formula (XVIII).

The reaction temperature is preferably from 0 to 200°C, and more preferably from 10 to 150°C.

### (Step V)

The step V is the step of preparing 3-N-methylamino-1-(2-thienyl)-1-propanol in the form of a racemate or an optically active substance, which is represented by the general formula (IV), by hydrolyzing the propanolamine derivative represented by the general formula (XVII) obtained in the step M or N in the presence of a base.

Examples of the base include hydroxides of alkali metal or their salts with weak acid; hydroxides of alkali earth metal or their salts with weak acid; and hydroxides of quaternary ammonium or their salts with weak acid. Specific examples thereof include sodium hydroxide, potassium hydroxide, sodium carbonate, sodium hydrogencarbonate, potassium carbonate, potassium hydrogencarbonate, cesium carbonate, tripotassium phosphate and tripotassium phosphate dihydrate. The amount of the base is preferably from 1 to 10 mols, and more preferably from 2 to 6 mols, per mol of the N-acyl compound represented by the general formula (XVII).

The solvent is not specifically limited as long as it does not exert an adverse influence on the reaction, and examples thereof include alcohols such as methanol, ethanol, propanol, isopropanol and butanol; amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methyl ether, diisopropyl ether, tetrahydrofuran, 1,4-dioxane, 1,2-dimethoxyethane and anisole; and water. These solvents can be used alone or in combination. The amount of the solvent is industrially preferably from about 50 to 3000 parts by weight based on 100 parts by weight of the N-acyl compound represented by the general formula (XVII) in view of operability and economic efficiency. When using the solvent other than water, a suitable amount of water can also be added thereto. The amount of water is preferably from 0 to 10000 mols, and more preferably from 2 to 6000 mols, per mol of the N-acyl compound represented by the general formula (XVII).

The reaction temperature is preferably from 0 to 200°C, and more preferably from 10 to 150°C.

3-N-methylamino-1(thienyl)-1-propanol can be purified by vacuum distillation. For example, the metal catalyst used in the asymmetric reduction cannot be easily removed by washing or recrystallization, but can be easily removed by distillation. This is an effective means because impurities such as coloranats can be easily removed.

The vacuum distillation can be conducted under reduced pressure within a range from 0.01 to 20 Torr, and preferably from 0.1 to 10 Torr in view of operability. The temperature is from 50 to 200°C, and is preferably from 70 to 180°C in view of operability.

3-N-methylamino-1(thienyl)-1-propanol can be purified by recrystallization. Impurities or colorants can be removed by recrystallization, and thus a compound with high purity can be obtained. Since very slight coloration, which could not be removed by distillation, can be completely removed, a compound with high purity can be obtained when recrystallization is conducted after the distillation operation.

The solvent, which can be used in the recrystallization, is not specifically limited as long as it does not exert an adverse influence on 3-N-methylamino-1(thienyl)-1-propanol, and examples thereof include methanol, ethanol, propanol, isopropanol and butanol; amides such as dimethylformamide and dimethylacetamide; pyrrolidones such as N-methyl-2-pyrrolidone; ketone·sulfoxides such as acetone, ethyl methyl ketone and dimethyl sulfoxide; aromatic hydrocarbons such as benzene, toluene, xylene and mesitylene; ethers such as butyl methyl ether, diisopropyl ether, tetrahydrofuran, 1,4-dioxane, 1,2-dimethoxyethane and anisole; hydrocarbon solvents such as hexane and heptane; and water. These solvents can be used alone or in combination. The amount of the solvent is industrially preferably from about 50 to 3000 parts by weight based on 100 parts by weight of 3-N-methylamino-1(thienyl)-1-propanol represented by the general formula (IX) in view of operability and economic efficiency. Among these solvents, toluene is particularly preferable in view of loss of the objective compound due to high dissolution and purification efficiency.

The recrystallization can be conducted at a dissolution temperature within a range from 20° to 200°C, and preferably from 40 to 150°C. Under cooling, a crystal is deposited from the solution prepared by dissolving with heating, and the cooling temperature can be set within a range from -30 to 60°C. The cooling temperature is preferably set to 30°C or lower in view of yield and is preferably set to -15°C or higher in view of achievement of high purity. When the crystal is not formed, seed crystal can also be appropriately added.

### Examples

Examples of the present invention will be described, but the present invention is not limited to the following

### Examples.

### (Example 1)

### Synthesis of (S)-N-[3-acetoxy-3-(2-thienyl)propyl]-N,N-dimethylamine

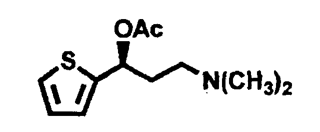

(S)-3-N,N-dimethylamino-1-(2-thienyl)-1-propanol (15 g, 81 mmol), triethylamine(9.0 g, 89 mmol) and chloroform (150 g) were charged in a flask and cooled to 5°C. To this was added dropwise acetyl chloride (10.8 g, 140 mmol) over 10 minutes. After reacting the mixture at 20°C for 2 hours, the reaction solution was washed in turn with saturated sodium bicarbonate water and saturated aqueous sodium chloride solution. The organic layer was concentrated under reduced pressure to obtain the objective compound as a pale yellow oily product. The amount of the objective compound was 16.9 g (yield: 92%).

The resulting compound was identified by the measurement by NMR. The results are shown below.
¹H NMR (270 MHz, DMSO-d6) 1.80-2.30 (m, 4H), 2.00 (s, 3H), 2.09 (s, 6H), 6.02 (t, 1H), 7.0-7.5 (3H)

### (Example 2)

### Synthesis of 2',2',2'-trichloroethyl (S)-N-[3-acetoxy-3-(2-thienyl)propyl]-N-methylcarbamate

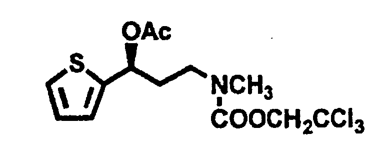

(S)-N-[3-acetoxy-3-(2-thienyl)propyl]-N,N-dimethylamine (8.0 g, 35 mmol) obtained in Example 1, PROTON SPONGE (trade mark) (1.7 g, 8 mmol), trichloroethylchloroformate (22.2 g, 105 mmol) and toluene (80 g) were charged in a flask and the mixture was stirred with heating at 70°C for 2 hours. After cooling to room temperature, methanol (6.0 g) and triethylamine (15.0 g, 150 mmol) were added, followed by stirring for 30 minutes. The reaction solution was washed in turn with 2N-hydrochloric acid and saturated aqueous sodium chloride solution, and then the organic layer was concentrated under reduced pressure and purified by column chromatography to obtain the objective compound as a pale yellow oily product. The amount of the objective compound was 11.9 g (yield: 87%).

The resulting compound was identified by the measurement by NMR. The results are shown below.
¹H NMR (270 MHz, DMSO-d6) 2.00 (s, 3H), 2.00-2.30 (m, 2H), 2.87-2.92 (d, 3H), 3.29-3.40 (m, 2H), 4.81 (s, 2H), 5.92-5.96 (m, H), 7.0-7.5 (3H)

### (Example 3)

### Synthesis of (S)-N-[3-hydroxy-3-(2-thienyl)propyl]-N-methylacetamide

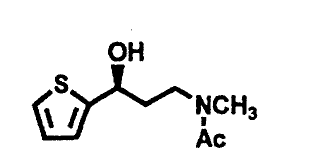

2',2',2'-trichloroethyl (S)-N-[3-acetoxy-3-(2-thienyl)propyl]-N-methylcarbamate (72 g, 0.18 mol) obtained in Example 2, Zn (120 g, 1.8 mol) and DMF (670 g) were charged in a flask and formic acid (36 g, 0.78 mol) was added under cooling. After reacting the mixture at 20°C for 2 hours, Zn was removed by filtration. The filtrate was concentrated and, after adjusting the pH to 12 by adding 28 wt% ammonia water, the filtrate was extracted with MTBE. The organic layer was concentrated under reduced pressure to obtain the objective compound as a pale yellow oily product. The amount of the objective compound was 35.5 g (yield: 90%).

The resulting compound was identified by the measurement by NMR. The results are shown below.
¹H NMR (270 MHz, CDCl₃) 1.84 (m, 1H), 1. 97 (m, 1H), 2.01 (s, 3H), 2.95 (s, 3H), 3.05 (m, 1H), 4.00 (m, 1H), 4.87 (dd 1H), 6.89-7.20 (3H)

### (Example 4)

### Synthesis of (S)-3-N-methylamino-1-(2-thienyl)-1-propanol

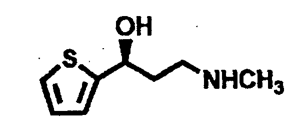

(S)-N-[3-hydroxy-3-(2-thienyl)propyl]-N-methylacetamide (21.3 g, 0.10 mol) obtained in Example 3, KOH (112 g, 2.0 mol) and methanol (448 g) were charged in a flask and heated at 70°C for 2 hours. The reaction solution was concentrated under reduced pressure and, after adding water (70 g), the solution was extracted three times with MTBE (200 g). The organic layer was washed with 10 wt% aqueous sodium chloride solution and then replaced with toluene and concentrated. After the deposited solid was removed by filtration and the filtrate was concentrated, n-heptane was added and the deposited solid was filtered with suction. The resulting solid was washed with 30 ml of a solution mixture of toluene/heptane (= 5/95(v/v)) and then dried under reduced pressure to obtain the objective compound as a white solid. The amount of the objective compound was 11.6 g (yield: 68%).

The resulting compound was identified by the measurement by NMR. The results are shown below.
¹H NMR (270 MHz, CDCl₃) 1.82-2.02 (m, 2H), 2.43 (s, 3H), 2.81-2.99 (m, 2H), 5.20 (m, 1H), 6.89-7.20 (3H)

### (Example 5)

### Synthesis of 3-(N-benzylmethylamino)-1-(2-thienyl)-1-propanone hydrochloride

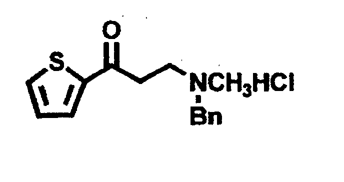

N-benzylmethylamine (36.9 g, 0.30 mmol) was dissolved in ethanol (40 ml) and a hydrochloride was formed by using 37% wt% hydrochloric acid (30.0 g, 0.30 mmol). To the solution, acetylthiophene (30 g,0.24 mmol), paraformaldehyde (10.8 g, 0.34 mmol), ethanol (20 ml) and 37 wt% hydrochloric acid (1.2 g, 0.01 mmol) were added and the mixture was heated under reflux at 80°C for 4 hours. After the reaction solution was cooled to room temperature, the deposited crystal was filtered and washed with ethanol. The crystal was dried at room temperature under reduced pressure to obtain the objective compound as a white crystal. The amount of the objective compound was 57.7 g (yield: 81.3%).

### (Example 6)

### (2) Synthesis of (RS)-3-N-methyl-N-benzylamino-1-(2-thienyl)-1-propanol

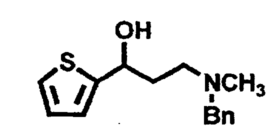

3-(N-benzylmethylamino)-1-(2-thienyl)-1-propanone hydrochloride (15.0 g, 50.7 mmol) obtained in Example 5 was suspended in methanol (104 ml) and the hydrochloride was neutralized by adding dropwise a solution prepared by dissolving sodium hydroxide (2.0 g, 50.7 mmol) in methanol (46 ml) was added dropwise under ice cooling. Then, sodium borohydride (3.8 g, 101.4 mmol) was added and the mixture was stirred under ice cooling for 2 hours. After the reaction solution was concentrated under reduced pressure, the residue was extracted with ethyl acetate and washed with saturated aqueous sodium chloride solution (150 ml). The organic layer was concentrated under reduced pressure to obtain the objective compound as an oily product. The amount of the objective compound was 11.9 g (yield: 90.0%).

The resulting compound was identified by the measurement by NMR. The results are shown below.
¹HNMR (270MHz, CDCl₃) 1.99 (dd, 2H), 2.24 (s, 3H), 2.73 (m, 2H), 3.54 (dd, 2H), 5.15 (dd, 1H), 6.88-6.96 (m, 3H), 7.18-7.33 (m, 5H)

### (Example 7)

### (3) Synthesis of (RS)-N-[3-acetoxy-3-(2-thienyl)propyl]-N-methyl-N-benzylamine

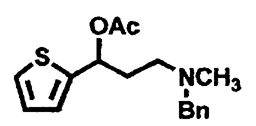

(RS)-3-N-methyl-N-benzylamino-1-(2-thienyl)-1-propanol(6.6 g,25.1 mmol) obtained in Example 6 was dissolved in THF (66 ml) and acetyl chloride (7.3 g, 101.3 mmol) was added dropwise under ice cooling, and then the mixture was stirred under ice cooling for one hour. The reaction solution was concentrated under reduced pressure, extracted with ethyl acetate and then washed with 4 wt% sodium hydrogencarbonate water (180 ml). The organic layer was concentrated under reduced pressure to obtain the objective compound as an oily product. The amount of the objective compound was 7.16 g (yield: 94.0%).

The resulting compound was identified by the measurement by NMR. The results are shown below.
¹HNMR (270MHz, CDCl₃) 1.93 (s, 3H), 2.09 (m, 2H), 2.15 (s, 3H), 2.37 (m, 2H), 3.42 (t, 2H), 6.11 (dd, 1H), 6.87-6.98 (m, 3H), 7.18-7.26 (m, 5H)

### (Example 8)

### (4) Synthesis of 2',2',2'-trichloroethyl (RS)-N-[3-acetoxy-3-(2-thienyl)propyl]-N-methylcarbamate

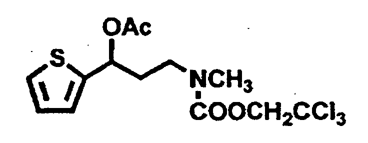

(RS)-N-[3-acetoxy-3-(2-thienyl)propyl]-N-methyl-N-benzylamine (1.1 g, 3.6 mmol) obtained in Example 7 was dissolved in acetonitrile (10 ml) and trichloroethyl chloroformate (0.5 ml, 3.6 mmol) was added dropwise at 5°C. After stirring at room temperature for 2 hours, the reaction solution was concentrated under reduced pressure to obtain the objective compound as an oily product. The amount of the objective compound was 1.7 g.

The resulting compound was identified by the measurement by NMR. The results are shown below.
¹HNMR (270MHz, CDCl₃) 2.14 (s, 3H), 2.22 (m, 2H), 2.96 (d, 3H), 3.37 (m, 2H), 4.12 (s, 2H), 6.03 (dd, 1H), 6.92-7.06 (m, 3H), 7.13-7.30 (m, 5H)

### (Example 9)

### (5) Synthesis of (RS)-N-[3-hydroxy-3-(2-thienyl)propyl]-N-methylacetamide

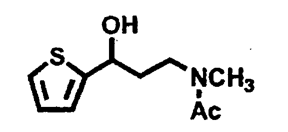

2',2',2'-trichloroethyl (RS)-N-[3-acetoxy-3-(2-thienyl)propyl]-N-methylcarbamate (0.5 g, 1.3 mmol) obtained in Example 8 was dissolved in acetic acid (5 ml) and Zn (0.2 g) was suspended, and then the mixture was stirred at room temperature for 5 hours. The reaction solution was filtered and the filtrate was concentrated under reduced pressure, and then the residue was extracted with ethyl acetate. The extract was washed with 4 wt% sodium hydrogencarbonate water (30 ml) and concentrated under reduced pressure to obtain the objective compound as an oily product. The amount of the objective compound was 0.5 g.

The resulting compound was identified by the measurement by NMR. The results are shown below.
¹HNMR (270MHz, CDCl₃) 2.10 (s, 3H), 2.03 (m, 2H), 2.95 (s, 3H), 3.04 (dt, 2H), 3.64 (s, 1H), 4.73 (dd, 1H), 6.86-6.97 (m, 3H)

### (Example 10)

### Synthesis of phenyl (RS)-N-[3-acetoxy-3-(2-thienyl)propyl]-N-methylcarbamate

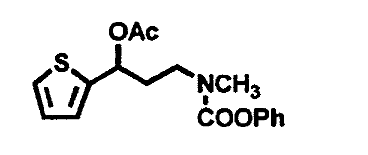

(RS)-N-[3-acetoxy-3-(2-thienyl)propyl]-N-methyl-N-benzylamine (3.9 g, 12.9 mmol) obtained in Example 7 was dissolved in acetonitrile (39 ml) and phenyl chloroformate (2.0 g, 12.9 mmol) was added dropwise at 5°C. After stirring the mixture at room temperature for one hour, the reaction solution was concentrated under reduced pressure to obtain the objective compound as an oily product. The amount of the objective compound was 5.2 g.

The resulting compound was identified by the measurement by NMR. The results are shown below.
¹HNMR (500MHz, CDCl₃) 1.98 (s, 3H), 2.23 (m, 2H), 3.03 (s, 3H), 3.44 (m, 2H), 6.10 (m, 1H), 6.91-7.10 (m, 3H), 7.17-7.38 (m, 5H)

### (Example 11)

### Synthesis of ethyl (RS)-N-[3-acetoxy-3-(2-thienyl)propyl]-N-methylcarbamate

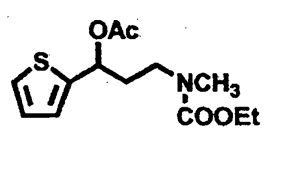

(RS)-N-[3-acetoxy-3-(2-thienyl)propyl]-N-methyl-N-benzylamine (1.0 g, 3.3 mmol) obained in Example 7 was dissolved in acetonitrile (10 ml) and ethyl chloroformate (0.4 g, 3.3 mmol) was added dropwise at 5°C. After stirring the mixture at room temperature for 2 hours, the reaction solution was concentrated under reduced pressure to obtain the objective compound as an oily product. The amount of the objective compound was 1.1 g.

The resulting compound was identified by the measurement by NMR. The results are shown below.
¹HNMR (270MHz, CDCl₃) 1.13 (t, 3H), 1.94 (s, 3H), 2.08 (m, 2H), 2.77 (s, 3H), 3.22 (m, 2H), 4.00 (dd, 2H), 5.92 (dd, 1H), 6.85 (dd, 2H), 6.97 (m, 1H)

### (Example 12)

### (8) Synthesis of phenyl (RS)-N-[3-phenyloxycarbonyloxy-3-(2-thienyl)propyl]-N-methylcarbamate

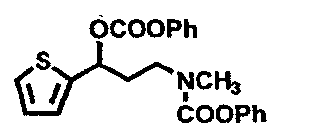

(RS)-3-N,N-dimethylamino-1(2-thienyl)-1-propanol (10 g, 54.0 mmol) was dissolved in acetonitrile (100 ml) and triethylamine (5.5 g, 54.0 mmol) was added under ice cooling. Furthermore, phenyl chloroformate (21.1 g,134.9 mmol) was added dropwise at 5°C and the mixture was stirred at room temperature for 2 hours. After the completion of the reaction, an aqueous 2 wt% sodium hydroxide solution (356 g) was added, followed by stirring under ice cooling for 0.5 hours. The reaction solution was separated between layers and the organic layer was concentrated under reduced pressure to obtain the objective compound as an oily product. The amount of the objective compound was 25.5 g.

The resulting compound was identified by the measurement by NMR. The results are shown below.
¹HNMR (270MHz, CDCl₃) 2.31 (m, 2H), 3.06 (s, 3H), 3.60 (m, 2H), 6.02 (m, 1H), 6.74-7.00 (m, 3H), 7.09-7.44 (m, 10H)

### (Example 13)

### (9) (RS)-3-N-methylamino-1-(2-thienyl)-1-propanol

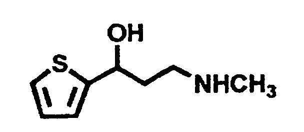

phenyl (RS)-N-[3-phenyloxycarbonyloxy-3-(2-thienyl)propyl]-N-methylcarbamate (50.2 mmol) obtained in Example 12 was dissolved in a 24 wt% potassium hydroxide-methanol solution (231.7 g) and the solution was heated under reflux at 80°C for 2 hours. The reaction solution was concentrated under reduced pressure and, after removing a salt with toluene, n-heptane was added to obtain the objective compound as a white solid. The amount of the objective compound was 0.9 g (yield: 44.4%).

The resulting compound was identified by the measurement by NMR. The results are shown below.
¹HNMR (270MHz, CDCl₃) 1.92 (m, 2H), 2.43 (s, 3H), 2.90 (m, 2H), 3.20 (s, 2H), 5.18 (m, 1H), 6.90 (m, 2H), 7.20 (m, 1H)

### (Example 14)

### Synthesis of (S)-3-N-methyl-N-benzylamino-1-(2-thienyl)-1-propanol

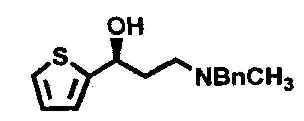

A 0.5M 2-propanol solution (40 µL) containing potassium hydroxide dissolved therein, (R,R)-diphenylethylenediamine (2.1 mg,0.01 mmol), 3-(N-benzylmethylamino)-1-(2-thienyl)-1-propanone (873 mg, 5.0 mmol) obtained in Example 5 and 2-propanol (3 ml) were charged in a Schlenk reaction tube under an argon gas flow and, after deaeration and replacement by argon, RuCl₂ ((R)-BINAP)(dmf)n (9.6 mg,0.01 mmol) was added to prepare a reaction solution. The solution was completely dissolved by repeatedly conducting the deaeration and replacement by argon. The solution was transferred to a 100 ml glass autoclave and hydrogen was introduced to a predetermined pressure, thereby initiating the reaction. After stirring at 28°C for 6 hours and returning to normal temperature, the objective compound was obtained.

Optical purity of the resulting compound was determined by the HPLC process using an optically active column. As a result, optical purity was high such as 96%ee.

### (Example 15)

### Synthesis of (S)-N-[3-acetoxy-3-(2-thienyl)propyl]-N-methyl-N-benzylamine

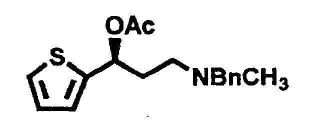

In the same manner as in Example 7, except that (S)-3-N-methyl-N-benzylamino-1-(2-thienyl)-1-propanol obtained in Example 14 was used in place of (RS)-3-N-methyl-N-benzylamino-1-(2-thienyl)-1-propanol, the titled compound was obtained. The yield was 96%.

### (Example 16)

### Synthesis of phenyl (S)-N-[3-acetoxy-3-(2-thienyl)propyl]-N-methylcarbamate

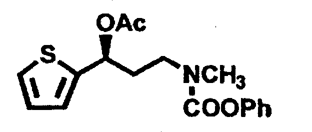

In the same manner as in Example 10, except that (S)-N-[3-acetoxy-3-(2-thienyl)propyl]-N-methyl-N-benzylamine obtained in Example 15 was used in place of (RS)-N-[3-acetoxy-3-(2-thienyl)propyl]-N-methyl-N-benzylamine, the titled compound was obtained. The yield was 92%.

### (Example 17)

### Synthesis of phenyl (S)-N-[3-phenyloxycarbonyloxy-3-(2-thienyl)propyl]-N-methylcarbamate

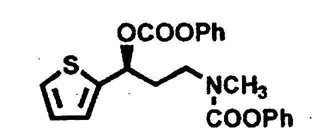

In the same manner as in Example 12, except that (S)-3-N,N-dimethylamino-1(2-thienyl)-1-propanol was used in place of (RS)-3-N,N-dimethylamino-1(2-thienyl)-1-propanol, the titled compound was obtained. The yield was 58%.

### (Example 18)

### Synthesis of (S)-3-N-methylamino-1-(2-thienyl)-1-propanol

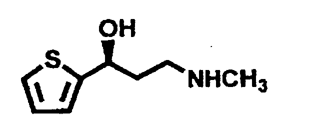

(S)-3-N,N-dimethylamino-1(2-thienyl)-1-propanol (55 g) and triethylamine (30 g) were added to MTBE (618 g) and phenyl chloroformate (116 g) was added dropwise while stirring at -5°C. The reaction solution was stirred at room temperature for 4 hours and 2 wt% sodium hydroxide (1955 g) was added dropwise while maintaining at 5°C or lower, followed by stirring for 30 minutes. The aqueous layer was removed and a solution mixture of potassium hydroxide (333 g)/methanol (1000 g) was added to the organic layer, followed by refluxing under stirring for 2 hours. The reaction solution was concentrated under reduced pressure and, after adding water (381 g) to the residue, the solution was extracted three times with MTBE (762 g). The organic layers were combined, washed with water (190 g) and then mixed with toluene (1434 g). The solvent was distilled off under reduced pressure until the amount of the residue reached 568 g, and then insolubles were removed by filtration. The filtrate was concentrated under reduced pressure and heptane (1007 g) was added, followed by stirring at -15°C to obtain the objective compound as a pale yellow solid. The amount of the objective compound was 28 g.

### (Example 19)

### Synthesis of isopropyl (RS)-N-[3-isopropyloxycarbonyloxy-3-(2-thienyl)propyl]-N-methylcarbamate

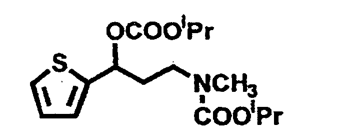

(R,S)-3-N,N-dimethylamino-1-(2-thienyl)-1-propanol (0.93 g, 5 mmol), triethylamine (0.66 g, 6.5 mmol) and THF (9.3 g) were charged in a flask and then cooled to 5°C. Then, isopropyl chloroformate (2.14 g, 17.5 mmol) was added dropwise over 10 minutes. After the mixture was reacted at 20°C for 2 hours, the reaction solution was added dropwise in a 5 wt% sodium hydroxide solution containing MTBE dissolved therein and the organic layer was extracted. The organic layer was washed with saturated aqueous sodium chloride solution and then concentrated under reduced pressure to obtain the objective compound as a pale yellow oily product. The amount of the objective compound was 1.80 g.

The resulting compound was identified by the measurement by NMR. The results are shown below.
¹H NMR (270 MHz, CDCl₃) 1.07-1.34 (m, 12H), 2.15-2.30 (m, 2H), 2.85(s, 3H), 3.18-3.30 (m, 2H), 4.79-4.90 (2H), 5.79-5.84 (m, 1H), 6.9-7.3 (3H)

### (Example 20)

### Synthesis of isobutyl (RS)-N-[3-isobutyloxycarbonyloxy-3-(2-thienyl)propyl]-N-methylcarbamate

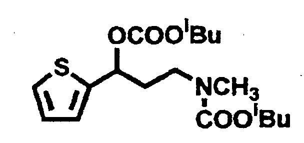

(R,S)-3-N,N-dimethylamino-1-(2-thienyl)-1-propanol (0.93 g, 5 mmol), triethylamine (0.66 g, 6.5 mmol) and THF (9.3 g) were charged in a flask and then cooled to 5°C. Then, isobutyl chloroformate (2.39 g, 17.5 mmol) was added dropwise over 10 minutes. After the mixture was reacted at 20°C for 2 hours, the reaction solution was added dropwise in a 5 wt% by sodium hydroxide solution containing MTBE dissolved therein and the organic layer was extracted. The organic layer was washed with saturated aqueous sodium chloride solution and then concentrated under reduced pressure to obtain the objective compound as a pale yellow oily product. The amount of the objective compound was 1.92 g.

The resulting compound was identified by the measurement by NMR. The results are shown below.
¹H NMR (270 MHz, CDCl₃) 0.88-0.96 (m, 12H), 1.82-1.95 (m, 2H), 2.10-2.30 (m, 2H), 2.87 (s, 3H), 3.18-3.40 (m, 2H), 3.80-3.88 (4H), 5.80-5.85 (m, 1H), 6.9-7.3 (3H)

### (Example 21)

### Synthesis of isobutyl (RS)-N-[3-isobutyloxycarbonyloxy-3-(2-thienyl)propyl]-N-methylcarbamate

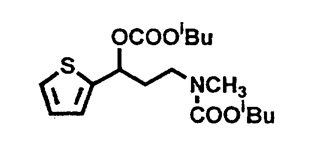

(R,S)-3-N,N-dimethylamino-1-(2-thienyl)-1-propanol (55.6 g, 0.30 mol), triethylamine (39.5 g, 0.39 mol) and toluene (278 g) were charged in a flask and then cooled to 5°C. Then, isobutyl chloroformate (123 g, 0.90mol) was added dropwise so that the inner temperature did not exceed 15°C. After the mixture was reacted at 25°C for 6 hours, an aqueous 10 wt% sodium hydroxide solution (360 g, 0.90 mmol) was added to the reaction solution and the organic layer was extracted. The organic layer was concentrated under reduced pressure to obtain the objective compound as a pale yellow oily product. The amount of the objective compound was 142 g and the yield of the objective compound was 96.0%. The yield of the objective compound was determined by high-performance liquid chromatography.

### (Example 22)

### Synthesis of (S)-3-N-methylamino-1(thienyl)-1-propanol

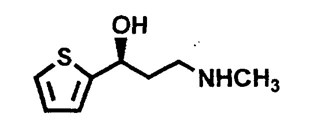

(S)-3-N,N-dimethylamino-1-(2-thienyl)-1-propanol (50.0 g, 0.27 mol), triethylamine (35.5 g, 0.35 mmol) and toluene (249 g) were charged in a flask, and then isobutyl chloroformate (110.5 g, 0.81 mmol) was added dropwise so that the inner temperature became 20 to 30°C. After the mixture was reacted at 25°C for 6 hours, an aqueous 10 wt% NaOH solution (324 g, 0.81 mmol) was added dropwise to the reaction solution and the organic layer was extracted. The organic layer was concentrated under reduced pressure to obtain 125.6 g of a pale yellow oily product. To this was added a 25 wt% potassium hydroxide-methanol solution (603 g, 2.67 mol), followed by heating under reflux at the inner temperature of 75°C for 3.5 hours. After the reaction solution was concentrated under reduced pressure until the amount reached 447 g, water (303 g) was added and the solution was replaced and concentrated until the inner amount reached 394 g. To the solution was added toluene (202 g), and then the organic layer was extracted. The organic layer was concentrated under reduced pressure to obtain 54.7 g of (S)-3-N-methylamino-1(thienyl)-1-propanol as a pale yellow solid. The pale yellow solid was subjected to vacuum distillation to obtain 41.5 g of a fraction at a vapor phase temperature within a range from 100 to 130°C under reduced pressure of 2 to 4 Torr. The fraction was mixed with toluene (103 g), dissolved with heating at 60°C and then cooled to 30°C. Then, a seed crystal of (S)-3-N-methylamino-1(thienyl)-1-propanol was added to deposit a crystal. After cooling to 5°C, the crystal was filtered with suction and washed with toluene (20.8 g) cooled to 5°C. The crystal was dried under reduced pressure to obtain 36.8 g of (S)-3-N-methylamino-1(thienyl)-1-propanol as a colorless crystal (yield: 79.6%).

### (Example 23)

### Synthesis of (S)-3-N,N-dimethylamino-1-(2-thienyl)-1-propanol

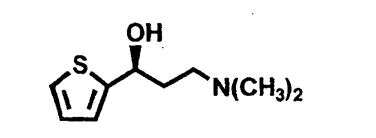

trans-RuCl₂((R)-xylBINAP)((R)-DAIPEN) (30.5 mg, 0.025 mmol), a 2-propanol solution (100 ml), a t-butanol solution (7.5 ml, 7.5 mmol) of 1.0M t-butoxy potassium and 3-(N-dimethylamino)-1-(2-thienyl)-1-propanone (22.9 g, 0.125 mol) were charged in a glass autoclave and, after repeatedly conducting deaeration and replacement by argon, hydrogen was introduced to a predetermined pressure, thereby to initiate the reaction. After stirring at 28°C for 6 hours, the reaction solution was returned to normal temperature and normal pressure. After the reaction solution was concentrated, heptane was added and the deposited solid was filtered with suction. The resulting solid was dried under reduced pressure to obtain the objective compound. The amount of the objective compound was 18.5 g (yield: 80.0%). Optical purity of the resulting compound was determined by the HPLC process using an optically active column. As a result, optical purity was high such as 99%ee.

(R)-DAIPEN denotes (R)-1-isopropyl-2,2-di(p-methoxyphenyl)ethylenediamine, and xylBINAP denotes 2,2'-bis(di-3,5-xylylphosphino)-1,1'-binaphthyl.

### INDUSTRIAL APPLICABILITY

As described in detail above, according to the present invention, it is made possible to provide means for preparing a racemate or an optically active substance (S- or R-isomer) of 3-N-methylamino-1-(2-thienyl)-1-propanol in a simple manner at low cost and in high yield.

## Claims

1. A propanolamine derivative represented by the following general formula (I): 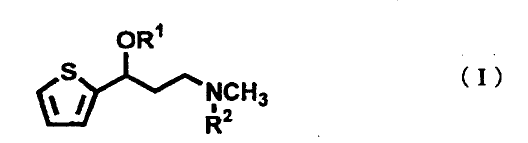 wherein R¹ represents any of a hydrogen atom, an acyl group having 1 to 8 carbon atoms, an alkyloxycarbonyl group having 1 to 8 carbon atoms which may have a substituent and a phenyloxycarbonyl group which may have a substituent, and R² represents any of a hydrogen atom, an alkyl group having 1 to 8 carbon atoms, a benzyl group which may have a substituent, an acyl group having 1 to 8 carbon atoms, an alkyloxycarbonyl group having 1 to 8 carbon atoms which may have a substituent and a phenyloxycarbonyl group which may have a substituent, with the exception that R¹ is a hydrogen atom and R² is a methyl group or a hydrogen atom.

2. The propanolamine derivative according to claim 1, which is the S-isomer.

3. The propanolamine derivative according to claim 1,
wherein R¹ and R² each independently represents an alkyloxycarbonyl group having 1 to 8 carbon atoms which may have a substituent, or a phenyloxycarbonyl group which may have a substituent.

4. The propanolamine derivative according to claim 3,
wherein R¹ and R² each independently represents a functional group represented by the following general formula (II):
**-COOCH**_{**2**}**CH**_{**m**}**X**_{**n**} (II)
wherein X represents a halogen atom, m and n each independently represents an integer of 0 to 3 and the sum of them is 3.

5. The propanolamine derivative according to claim 4,
wherein R¹ and R² represent a 2,2,2-trichloroethyloxycarbonyl group.

6. The propanolamine derivative according to claim 3,
wherein R¹ and R² represent a phenyloxycarbonyl group.

7. The propanolamine derivative according to claim 3,
wherein R¹ and R² represent an isopropyloxycarbonyl group.

8. The propanolamine derivative according to claim 3,
wherein R¹ and R² represent an isobutyloxycarbonyl group.

9. The propanolamine derivative according to claim 3,
wherein R¹ and R² represent an ethyloxycarbonyl group.

10. The propanolamine derivative according to claim 1,
wherein R¹ is a hydrogen atom and R² is an acyl group having 1 to 8 carbon atoms.

11. The propanolamine derivative according to claim 10,
wherein R² is an acetyl group.

12. The propanolamine derivative according to claim 1,
wherein R¹ is a hydrogen atom and R² is an alkyloxycarbonyl group having 1 to 8 carbon atoms which may have a substituent, or a phenyloxycarbonyl group which may have a substituent.

13. The propanolamine derivative according to claim 12,
wherein R² is a phenyloxycarbonyl group.

14. The propanolamine derivative according to claim 12,
wherein R² is a 2,2,2-trichloroethyloxycarbonyl group.

15. The propanolamine derivative according to claim 12,
wherein R² is an isopropyloxycarbonyl group.

16. The propanolamine derivative according to claim 12,
wherein R² is an isobutyloxycarbonyl group.

17. The propanolamine derivative according to claim 1,
wherein R¹ is an acyl group having 1 to 8 carbon atoms and R² is any of a hydrogen atom, an alkyl group having 1 to 8 carbon atoms, a benzyl group which may have a substituent, an alkyloxycarbonyl group having 1 to 8 carbon atoms which may have a substituent, a phenyloxycarbonyl group which may have a substituent and an acyl group having 1 to 8 carbon atoms.

18. The propanolamine derivative according to claim 17,
wherein R² is an alkyl group having 1 to 8 carbon atoms or a benzyl group which may have a substituent.

19. The propanolamine derivative according to claim 18,
wherein R² is a benzyl group which may have a substituent.

20. The propanolamine derivative according to claim 17,
wherein R² is an alkyloxycarbonyl group having 1 to 8 carbon atoms which may have a substituent or a phenyloxycarbonyl group which may have a substituent.

21. The propanolamine derivative according to claim 20,
wherein R² is a functional group represented by the following general formula (II):
**-COOCH**_{**2**}**CH**_{**m**}**X**_{**n**} (II)
wherein X represents a halogen atom, m and n each independently represents an integer of 0 to 3 and the sum of them is 3.

22. The propanolamine derivative according to claim 21,
wherein R² is a 2,2,2-trichloroethyloxycarbonyl group.

23. The propanolamine derivative according to claim 20,
wherein R² is a phenyloxycarbonyl group.

24. The propanolamine derivative according to claim 20,
wherein R² is an isopropyloxycarbonyl group.

25. The propanolamine derivative according to claim 20,
wherein R² is an isobutyloxycarbonyl group.

26. The propanolamine derivative according to claim 17,
wherein R¹ and R² each independently represents an acyl group having 1 to 8 carbon atoms.

27. The propanolamine derivative according to claim 1,
wherein R¹ is a hydrogen atom and R² is a benzyl group which may have a substituent.

28. A process for preparing 3-N-methylamino-1-(2-thienyl)-1-propanol, which comprises preparing 3-N-methylamino-1-(2-thienyl)-1-propanol represented by the following general formula (IV): 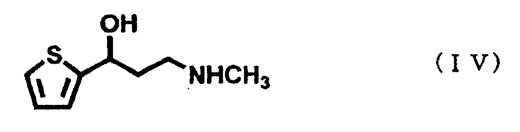 using or via the propanolamine derivative of claim 1.

29. A process for preparing 3-N-methylamino-1-(2-thienyl)-1-propanol represented by the following general formula (IV) : 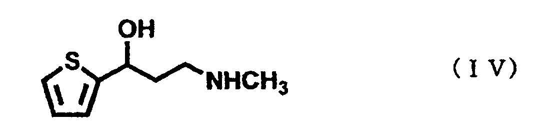 which comprises the step of eliminating the substituent R³ of a propanolamine derivative represented by the following general formula (III): 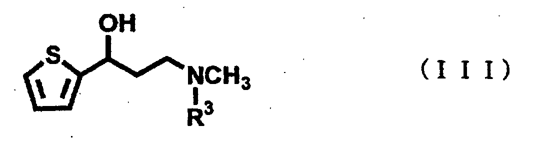 wherein R³ represents an alkyl group having 1 to 8 carbon atoms which may have a substituent or a benzyl group which may have a substituent.

30. The process for preparing 3-N-methylamino-1-(2-thienyl)-1-propanol according to claim 29, wherein both of the propanolamine derivatives represented by the general formulas (III) and (IV) are the S-isomer.

31. A process for preparing 3-N-methylamino-1-(2-thienyl)-1-propanol, which comprises the steps (E) of reacting acetylthiophene, a dialkylamine represented by the following general formula (V):
**HNCH**_{**3**}**R**^{**3**} (V)
wherein R³ represents an alkyl group having 1 to 8 carbon atoms which may have a substituent or a benzyl group which may have a substituent, and formalin under acidic conditions to obtain a ketone compound represented by the following general formula (VI): 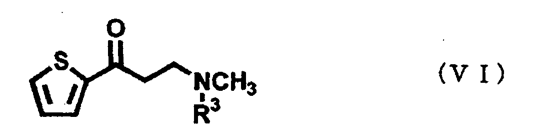 wherein R³ is as defined above;
the step (F) of reducing the ketone compound to obtain a propanolamine derivative represented by the following general formula (III): 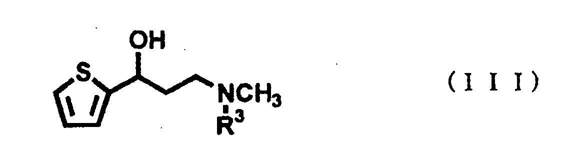 wherein R³ is as defined above; and
the step (S) of eliminating the substituent R³ of the propanolamine derivative represented by the general formula (III) to obtain 3-N-methylamino-1-(2-thienyl)-1-propanol represented by the following general formula (IV).

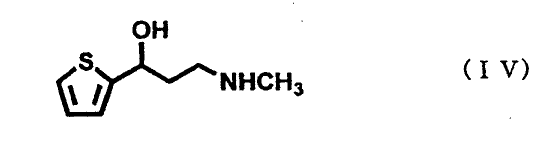

32. A process for preparing (S)-3-N-methylamino-1-(2-thienyl)-1-propanol, which comprises the step (E) of reacting acetylthiophene, a dialkylamine represented by the following general formula (V):
**HNCH**_{**3**}**R**^{**3**} (V)
wherein R³ represents an alkyl group having 1 to 8 carbon atoms which may have a substituent or a benzyl group which may have a substituent, and formalin under acidic conditions, to obtain a ketone compound represented by the following general formula (VI): 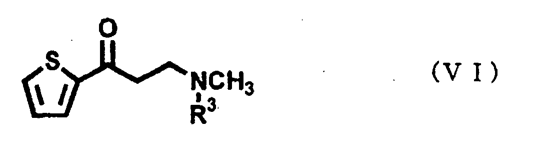 wherein R³ is as defined above;
the step (F) of reducing the ketone compound to obtain a propanolamine derivative represented by the following general formula (III): 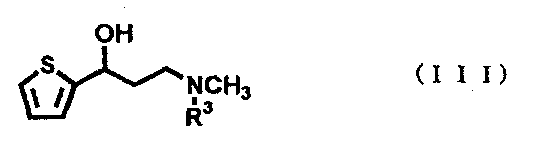 wherein R³ is as defined above;
the step (G) of optically resolving the propanolamine derivative represented by the general formula (III) using an optically active organic acid to obtain a propanolamine derivative in the form of the S-isomer represented by the following general formula (VII): 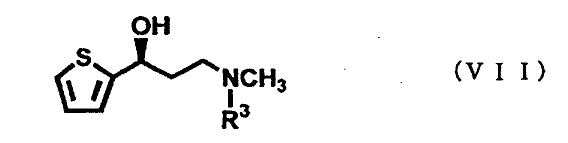 wherein R³ is as defined above; and
the step (I) of eliminating the substituent R³ of the propanolamine derivative in the form of the S-isomer to obtain (S)-3-N-methylamino-1-(2-thienyl)-1-propanol represented by the following general formula (VIII).

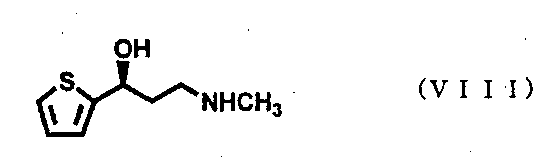

33. A process for preparing (S)-3-N-methylamino-1-(2-thienyl)-1-propanol, which comprises the step (E) of reacting acetylthiophene with a dialkylamine represented by the following general formula (V):
**HNCH**_{**3**}**R**^{**3**} (V)
wherein R³ represents an alkyl group having 1 to 8 carbon atoms which may have a substituent or a benzyl group which may have a substituent under acidic conditions to obtain a ketone compound represented by the following general formula (VI): 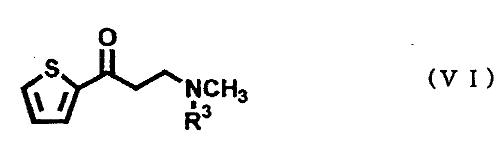 wherein R³ is as defined above;
the step (H) of reacting the ketone compound with hydrogen in the presence of an asymmetrical hydrogenation catalyst containing a transition metal, thereby conducting asymmetrical hydrogenation of the ketone compound to obtain a propanolamine derivative in the form of the S-isomer represented by the following general formula (VII): 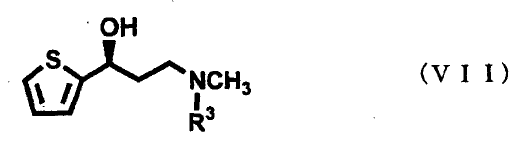 wherein R³ is as defined above; and
the step (I) of eliminating the substituent R³ of the propanolamine derivative in the form of the S-isomer to obtain (S)-3-N-methylamino-1-(2-thienyl)-1-propanol represented by the following general formula (VIII).

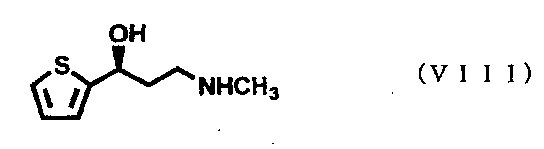

34. The process for preparing (S)-3-N-methylamino-1-(2-thienyl)-1-propanol according to claim 33, wherein, in the step (H), the asymmetrical hydrogenation of the ketone compound is conducted in the presence of a base and an optically active nitrogen-containing compound, in addition to the asymmetrical hydrogenation catalyst.

35. The process for preparing 3-N-methylamino-1-(2-thienyl)-1-propanol according to any one of claims 29 and 31 to 33, which comprises the step of reductively eliminating the substituent R³ of the propanolamine derivative represented by the general formula (III) or (VII).

36. The process for preparing 3-N-methylamino-1-(2-thienyl)-1-propanol according to any one of claims 29 and 31 to 33, which comprises the step of reacting the propanolamine derivative represented by the general formula (III) or (VII) with a chloroformic acid derivative represented by the following general formula (IX):
**CICOOR**^{**4**} (IX)
wherein R⁴ represents an alkyl group having 1 to 8 carbon atoms which may have a substituent or a phenyl group which may have a substituent, and hydrolyzing the intermediate without isolation to obtain 3-N-methylamino-1-(2-thienyl)-1-propanol.

37. The process for preparing 3-N-methylamino-1-(2-thienyl)-1-propanol according to any one of claims 29 and 31 to 33, which further comprises the step of reacting the propanolamine derivative represented by the general formula (III) or (VII) with a chloroformic acid derivative represented by the following general formula (X):
**CICOOCH**_{**2**}**CH**_{**m**}**X**_{**n**} (X)
wherein X represents a halogen atom, m and n each independently represents an integer of 0 to 3 and the sum of them is 3, and subjecting the intermediate to dehalogenation/alkyloxycarbonylation and hydrolysis without isolation to obtain 3-N-methylamino-1-(2-thienyl)-1-propanol.

38. The process for preparing 3-N-methylamino-1-(2-thienyl)-1-propanol according to any one of claims 29 and 31 to 33, which comprises the step (J) of reacting the propanolamine derivative represented by the general formula (III) or (VII) with a chloroformic acid derivative represented by the following general formula (IX):
**CICOOR**^{**4**} (IX)
wherein R⁴ represents an alkyl group having 1 to 8 carbon atoms which may have a substituent or a phenyl group which may have a substituent, to obtain a propanolamine derivative represented by the following general formula (XI): 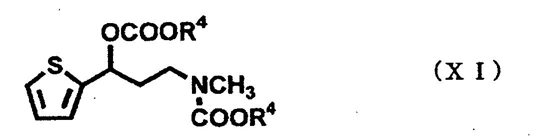 wherein R⁴ is as defined above; and
the step (R) of hydrolyzing the propanolamine derivative represented by the general formula (XI) to obtain 3-N-methylamino-1-(2-thienyl)-1-propanol.

39. The process for preparing 3-N-methylamino-1-(2-thienyl)-1-propanol according to any one of claims 29 and 31 to 33, which comprises the step (J) of reacting the propanolamine derivative represented by he general formula (III) or (VII) with a chloroformic acid derivative represented by the following general formula (IX):
**CICOOR**^{**4**} (IX)
wherein R⁴ represents an alkyl group having 1 to 8 carbon atoms which may have a substituent or a phenyl group which may have a substituent to obtain a propanolamine derivative represented by the following general formula (XI): 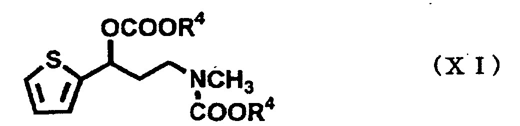 wherein R⁴ is as defined above;
the step (O) of treating the propanolamine derivative represented by the general formula (XI) with a base in the amount of 0.1 to 1.9 equivalents based on the compound, to obtain a propanolamine derivative represented by the following general formula (XII): 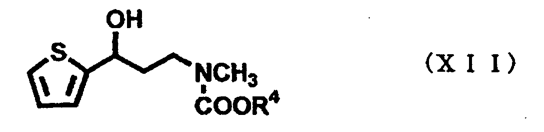 wherein R⁴ is as defined above; and
the step (Q) of hydrolyzing the propanolamine derivative represented by the general formula (XII) to obtain 3-N-methylamino-1-(2-thienyl)-1-propanol.

40. The process for preparing 3-N-methylamino-1-(2-thienyl)-1-propanol according to any one of claims 29 and 31 to 33, which comprises the step (K) of reacting the propanolamine derivative represented by the general formula (III) or (VII) with an alkylcarboxylic acid chloride or alkylcarboxylic anhydride represented by the following general formula (XIII):
**CICOR**^{**6**} (XIII)
wherein R⁶ represents an alkyl group having 1 to 8 carbon atoms or a phenyl group, or the general formula (XIV):
**(R**^{**6**}**CO)**_{**2**}**O** (XIV)
wherein R⁶ is as defined above, to obtain a propanolamine derivative represented by the following general formula (XV): 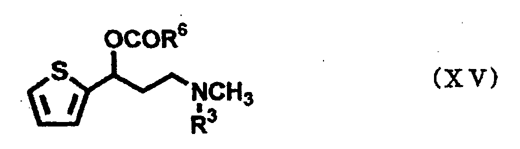 wherein R³ and R⁶ are as defined above;
the step (L) of reacting the propanolamine derivative represented by the general formula (XV) with a chloroformic acid derivative represented by the following general formula (IX):
**CICOOR**^{**4**} (IX)
wherein R⁴ represents an alkyl group having 1 to 8 carbon atoms which may have a substituent or a phenyl group which may have a substituent, to obtain a propanolamine derivative represented by the following general formula (XVI): 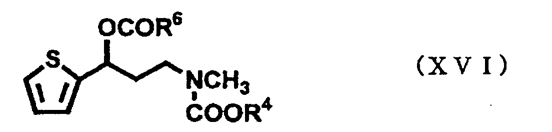 wherein R⁴ and R⁶ are as defined above); and
the step (T) of hydrolyzing the propanolamine derivative represented by the general formula (XVI) to obtain 3-N-methylamino-1-(2-thienyl)-1-propanol.

41. The process for preparing 3-N-methylamino-1-(2-thienyl)-1-propanol according to any one of claims 29 and 31 to 33, which comprises the step (K) of reacting the propanolamine derivative represented by the general formula (III) or (VII) with an alkylcarboxylic acid chloride or alkylcarboxylic anhydride represented by the following general formula (XIII):
**CICOR**^{**6**} (XIII)
wherein R⁶ represents an alkyl group having 1 to 8 carbon atoms or a phenyl group, or the general formula (XIV):
**(R**^{**6**}**CO)**_{**2**}**O** (XIV)
wherein R⁶ is as defined above, to obtain a propanolamine derivative represented by the following general formula (XV): 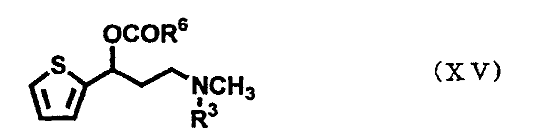 wherein R³ and R⁶ are as defined above;
the step (M) of reducing the propanolamine derivative represented by the general formula (XV) to obtain a propanolamine derivative represented by the following general formula (XVII): 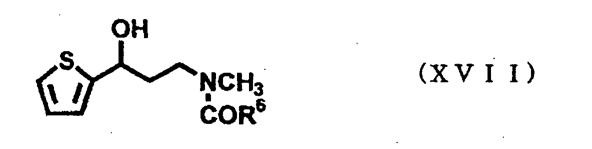 wherein R⁶ is as defined above; and
the step (V) of hydrolyzing the propanolamine derivative represented by the general formula (XVII) to obtain 3-N-methylamino-1-(2-thienyl)-1-propanol.

42. The process for preparing 3-N-methylamino-1-(2-thienyl)-1-propanol according to any one of claims 29 and 31 to 33, which comprises the step (K) of reacting a propanolamine derivative represented by the general formula (III) or (VII) with an alkylcarboxylic acid chloride or alkylcarboxylic anhydride represented by the following general formula (XIII):
**CICOR**^{**6**} (XIII)
wherein R⁶ represents an alkyl group having 1 to 8 carbon atoms or a phenyl group, or the general formula (XIV):
**(R**^{**6**}**CO)**_{**2**}**O** (XIV)
wherein R⁶ is as defined above, to obtain a propanolamine derivative represented by the following general formula (XV): 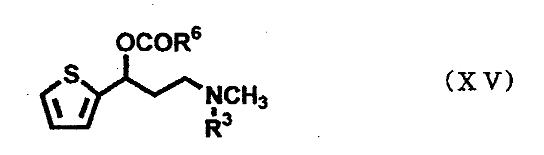 wherein R³ and R⁶ are as defined above;
the step (M) of reducing the propanolamine derivative represented by the general formula (XV) to obtain a propanolamine derivative represented by the following general formula (XVII): 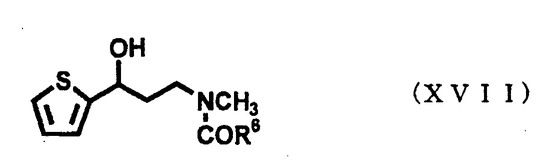 wherein R⁶ is as defined above;
the step (P) of reacting the propanolamine derivative represented by the general formula (XVII) with an alkylcarboxylic acid chloride or alkylcarboxylic anhydride represented by the general formula (XIII) or (XIV) to obtain a propanolamine derivative represented by the following general formula (XVIII): 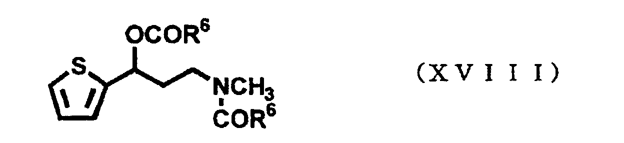 wherein R⁶ is as defined above; and
the step (U) of hydrolyzing the propanolamine derivative represented by the general formula (XVIII) to obtain 3-N-methylamino-1-(2-thienyl)-1-propanol.

43. The process for preparing 3-N-methylamino-1-(2-thienyl)-1-propanol according to any one of claims 29 and 31 to 33, which comprises the step (K) of reacting the propanolamine derivative represented by the general formula (III) or (VII) with an alkylcarboxylic acid chloride or alkylcarboxylic anhydride represented by the following general formula (XIII):
**CICOR**^{**6**} (XIII)
wherein R⁶ represents an alkyl group having 1 to 8 carbon atoms or a phenyl group, or the general formula (XIV):
**(R**^{**6**}**CO)**_{**2**}**O** (XIV)
wherein R⁶ is as defined above, to obtain a propanolamine derivative represented by the following general formula (XV): 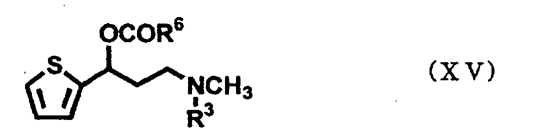 wherein R³ and R⁶ are as defined above;
the step (N) of reacting the propanolamine derivative represented by the general formula (XV) with a chloroformic acid derivative represented by the following general formula (X):
**CICOOCH**_{**2**}**CH**_{**m**}**X**_{**n**} (X)
wherein X represents a halogen atom, m and n each independently represents an integer of 0 to 3 and the sum of them is 3, to obtain a propanolamine derivative represented by the following general formula (XIX): 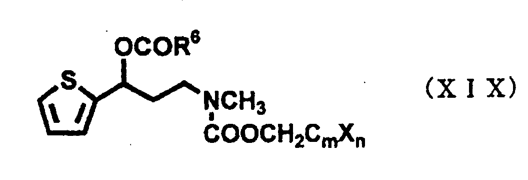 wherein R⁶, X, m and n are as defined above, and conducting the cleavage reaction of the urethane moiety and the transfer reaction of the acyl group of the propanolamine derivative represented by the general formula (XIX) to obtain a propanolamine derivative represented by the following general formula (XVII): 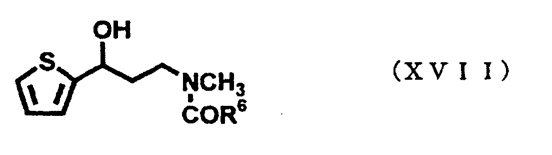 wherein R⁶ is as defined above; and
the step (V) of hydrolyzing the propanolamine derivative represented by the general formula (XVII) to obtain 3-N-methylamino-1-(2-thienyl)-1-propanol.

44. The process for preparing 3-N-methylamino-1-(2-thienyl)-1-propanol according to any one of claims 29 and 31 to 33, which comprises the step (K) of reacting the propanolamine derivative represented by the general formula (III) or (VII) with an alkylcarboxylic acid chloride or alkylcarboxylic anhydride represented by the following general formula (XIII):
**CICOR**^{**6**} (XIII)
wherein R⁶ represents an alkyl group having 1 to 8 carbon atoms or a phenyl group, or the general formula (XIV):
**(R**^{**6**}**CO)**_{**2**}**O** (XIV)
wherein R⁶ is as defined above, to obtain a propanolamine derivative represented by the following general formula (XV): 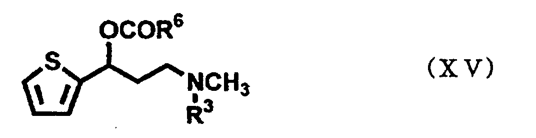 wherein R³ and R⁶ are as defined above;
the step (N) of reacting the propanolamine derivative represented by the general formula (XV) with a chloroformic acid derivative represented by the following general formula (X):
**CICOOCH**_{**2**}**CH**_{**m**}**X**_{**n**} (X)
wherein X represents a halogen atom, m and n each independently represents an integer of 0 to 3 and the sum of them is 3, to obtain a propanolamine derivative represented by the following general formula (XIX): 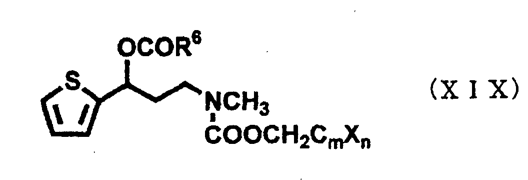 wherein R⁶, X, m and n are as defined above, and conducting the cleavage reaction of the urethane moiety and the transfer reaction of the acyl group of the propanolamine derivative represented by the general formula (XIX) to obtain a propanolamine derivative represented by the following general formula (XVII): 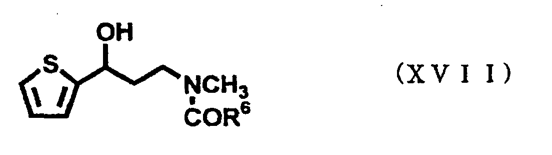 wherein R⁶ is as defined above;
the step (P) of reacting the propanolamine derivative with the general formula (XVII) with an alkylcarboxylic acid chloride or alkylcarboxylic anhydride represented by the general formula (XIII) or (XIV) to obtain a propanolamine derivative represented by the following general formula (XVIII): 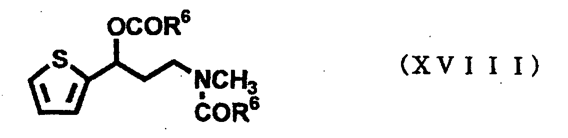 wherein R⁶ is as defined above; and
the step (U) of hydrolyzing the propanolamine derivative represented by the general formula (XVIII) to obtain 3-N-methylamino-1-(2-thienyl)-1-propanol.

45. A process for preparing a propanolamine derivative represented by the following general formula (XI): 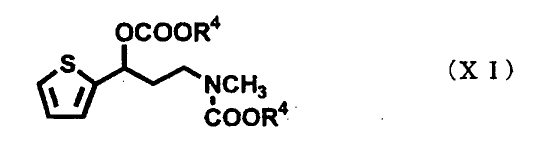 wherein R⁴ is as defined below, which comprises the step of reacting a propanolamine derivative represented by the following general formula (III): 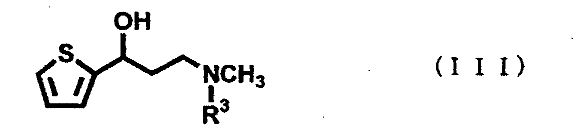 wherein R³ represents an alkyl group having 1 to 8 carbon atoms which may have a substituent or a benzyl group which may have a substituent, with a chloroformic acid derivative represented by the following general formula (IX):
**CICOOR**^{**4**} (IX)
wherein R⁴ represents an alkyl group having 1 to 8 carbon atoms which may have a substituent or a phenyl group which may have a substituent.

46. The process for preparing a propanolamine derivative according to claim 45, wherein R⁴ of the chloroformic acid derivative represented by the general formula (IX) is a phenyl group, an isopropyl group or an isobutyl group.

47. The process for preparing a propanolamine derivative according to claim 45, wherein both of the propanolamine derivatives represented by the general formulas (III) and (XI) are the S-isomer.

48. A process for preparing a propanolamine derivative represented by the following general formula (XII): 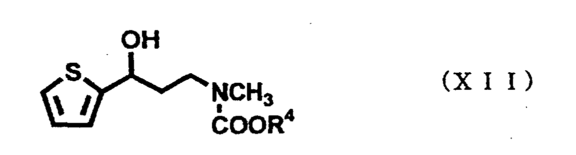 wherein R⁴ is as defined below, which comprises the step of reacting a propanolamine derivative represented by the following general formula (XI): 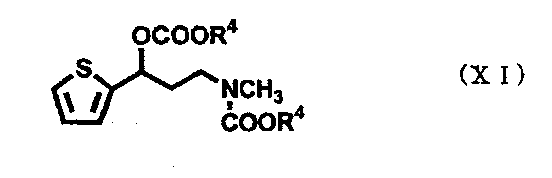 wherein R⁴ represents an alkyl group having 1 to 8 carbon atoms which may have a substituent or a phenyl group which may have a substituent, with a base.

49. The process for preparing a propanolamine derivative according to claim 48, wherein both of the propanolamine derivatives represented by the general formulas (XI) and (XII) are the S-isomer.

50. A process for preparing 3-N-methylamino-1-(2-thienyl)-1-propanol represented by the following general formula (IV): 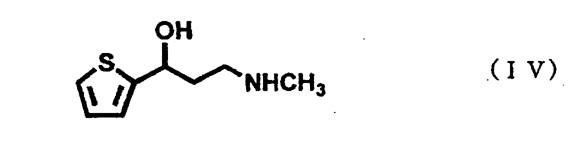 which comprises the step of hydrolyzing a propanolamine derivative represented by the following general formula (XII) : 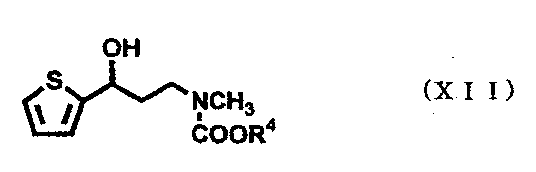 wherein R⁴ represents an alkyl group having 1 to 8 carbon atoms which may have a substituent or a phenyl group which may have a substituent.

51. The process for preparing 3-N-methylamino-1-(2-thienyl)-1-propanol according to claim 50, wherein both of the propanolamine derivatives represented by the general formulas (XII) and (IV) are the S-isomer.

52. A process for preparing 3-N-methylamino-1-(2-thienyl)-1-propanol represented by the following general formula (IV): 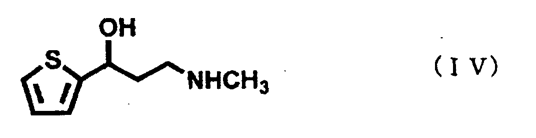 which comprises hydrolyzing a propanolamine derivative represented by the following general formula (XI): 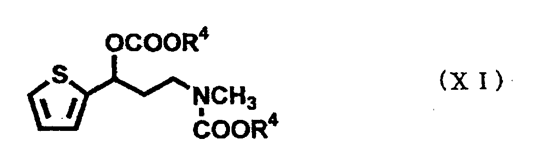 wherein R⁴ represents an alkyl group having 1 to 8 carbon atoms which may have a substituent or a phenyl group which may have a substituent.

53. The process for preparing 3-N-methylamino-1-(2-thienyl)-1-propanol according to claim 52, wherein both of the propanolamine derivatives represented by the general formulas (XI) and (IV) are the S-isomer.

54. A process for preparing a propanolamine derivative represented by the following general formula (XV): 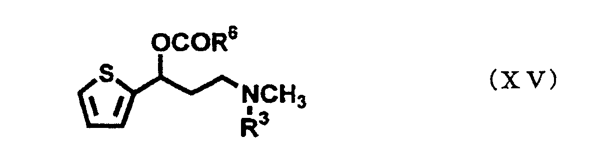 wherein R³ and R⁶ are as defined below, which comprises the step of reacting a propanolamine derivative represented by the following general formula (III): 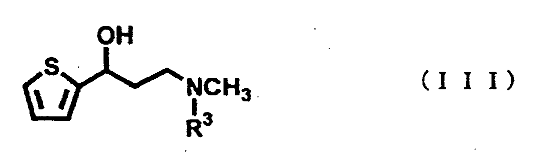 wherein R³ represents an alkyl group having 1 to 8 carbon atoms which may have a substituent or a benzyl group which may have a substituent, with an alkylcarboxylic acid chloride or alkylcarboxylic anhydride represented by the following general formula (XIII):
**CICOR**^{**6**} (XIII)
wherein R⁶ represents an alkyl group having 1 to 8 carbon atoms or phenyl group, or the general formula (XIV):
**(R**^{**6**}**CO)**_{**2**}**O** (XIV)
wherein R⁶ is as defined above.

55. The process for preparing a propanolamine derivative according to claim 54, wherein both of the propanolamine derivatives represented by the general formulas (III) and (XV) are the S-isomer.

56. A process for preparing a propanolamine derivative represented by the following general formula (XVI): 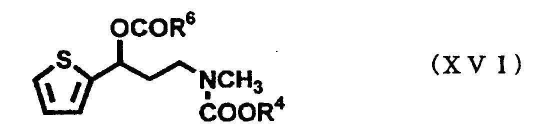 wherein R⁴ and R⁶ are as defined below, which comprises the step of reacting a propanolamine derivative represented by the following general formula (XV): 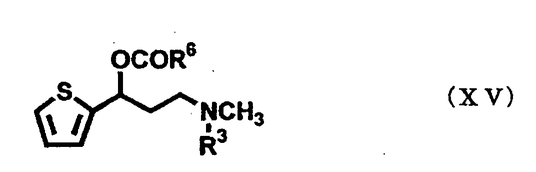 wherein R³ represents an alkyl group having 1 to 8 carbon atoms which may have a substituent or a benzyl group which may have a substituent, and R⁶ represents an alkyl group having 1 to 8 carbon atoms or phenyl group, with a chloroformic acid derivative represented by the following general formula (IX):
**CICOOR**^{**4**} (IX)
wherein R⁴ represents an alkyl group having 1 to 8 carbon atoms which may have a substituent or a phenyl group which may have a substituent.

57. The process for preparing a propanolamine derivative according to claim 56, wherein both of the propanolamine derivatives represented by the general formulas (XV) and (XVI) are the S-isomer.

58. A process for preparing a propanolamine derivative represented by the following general formula (XVII): 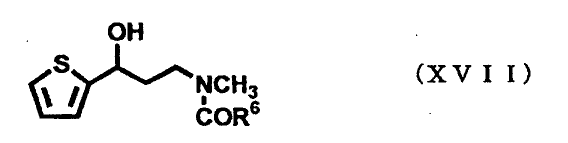 wherein R⁶ is as defined below, which comprises the step of reducing a propanolamine derivative represented by the following general formula (XV): 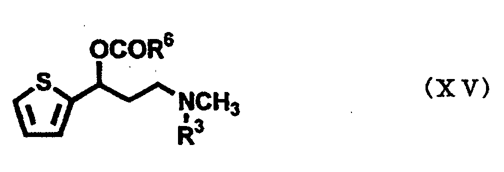 wherein R³ represents an alkyl group having 1 to 8 carbon atoms which may have a substituent or a benzyl group which may have a substituent, and R⁶ represents an alkyl group having 1 to 8 carbon atoms or a phenyl group.

59. The process for preparing a propanolamine derivative according to claim 58, wherein both of the propanolamine derivatives represented by the general formulas (XV) and (XVII) are the S-isomer.

60. A process for preparing 3-N-methylamino-1-(2-thienyl)-1-propanol represented by the following general formula (IV): 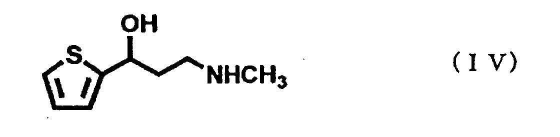 which comprises the step of hydrolyzing a propanolamine derivative represented by the following general formula (XVII): 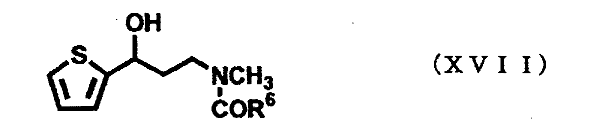 wherein R⁶ represents an alkyl group having 1 to 8 carbon atoms or a phenyl group.

61. The process for preparing 3-N-methylamino-1-(2-thienyl)-1-propanol according to claim 60, wherein both of the propanolamine derivatives represented by the general formulas (XVII) and (IV) are the S-isomer.

62. A process for preparing a propanolamine derivative represented by the following general formula (XVIII): 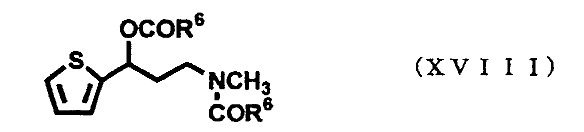 wherein R⁶ is as defined below, which comprises the step of reacting a propanolamine derivative represented by the following general formula (XVII): 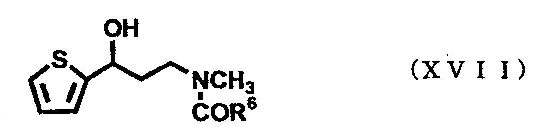 wherein R⁶ represents an alkyl group having 1 to 8 carbon atoms or phenyl group, with an alkylcarboxylic acid chloride or alkylcarboxylic anhydride represented by the following general formula (XIII):
**CICOR**^{**6**} (XIII)
wherein R⁶ is as defined above, or the general formula (XIV):
**(R**^{**6**}**CO)**_{**2**}**O** (XIV)
wherein R⁶ is as defined above.

63. The process for preparing a propanolamine derivative according to claim 62, wherein both of the propanolamine derivatives represented by the general formulas (XVII) and (XVIII) are the S-isomer.

64. A process for preparing 3-N-methylamino-1-(2-thienyl)-1-propanol represented by the following general formula (IV) : 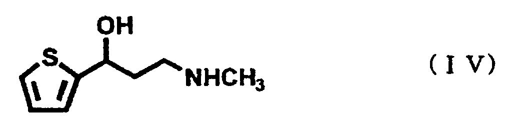 which comprises the step of hydrolyzing a propanolamine derivative represented by the following general formula (XVIII): 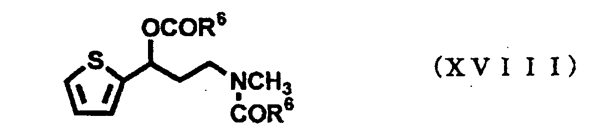 wherein R⁶ represents an alkyl group having 1 to 8 carbon atoms or a phenyl group.

65. The process for preparing 3-N-methylamino-1-(2-thienyl)-1-propanol according to claim 64, wherein both of the propanolamine derivatives represented by the general formulas (XVIII) and (IV) are the S-isomer.

66. A process for preparing a propanolamine derivative represented by the following general formula (XVII): 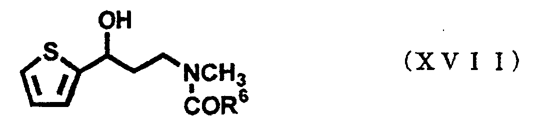 wherein R⁶ is as defined below, which comprises the step of conducting the cleavage reaction of the urethane moiety and the transfer reaction of the acyl group of the propanolamine derivative represented by the general formula (XIX): 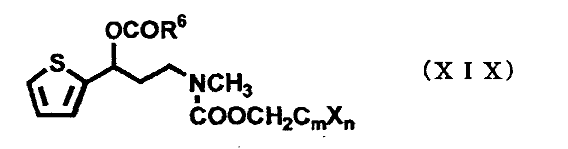 wherein R⁶ represents an alkyl group having 1 to 8 carbon atoms or phenyl group, X represents a halogen atom, m and n each independently represents an integer of 0 to 3 and the sum of them is 3.

67. The process for preparing a propanolamine derivative according to claim 66, wherein both of the propanolamine derivatives represented by the general formulas (XIX) and (XVII) are the S-isomer.

68. A process for preparing 3-N-methylamino-1-(2-thienyl)-1-propanol represented by the following general formula (IV): 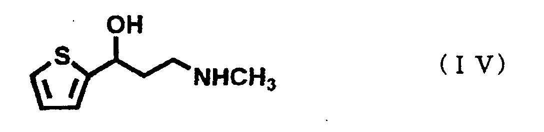 which comprises the step of hydrolyzing a propanolamine derivative represented by the following general formula (XVI) : 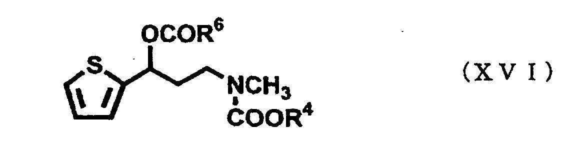 wherein R⁴ represents an alkyl group having 1 to 8 carbon atoms which may have a substituent or a phenyl group which may have a substituent, and R⁶ represents an alkyl group having 1 to 8 carbon atoms or a phenyl group.

69. The process for preparing 3-N-methylamino-1-(2-thienyl)-1-propanol according to claim 68, wherein both of the propanolamine derivatives represented by the general formulas (XVI) and (IV) are the S-isomer.

70. A process for preparing a propanolamine derivative in the form of the S-isomer represented by the following general formula (VII): 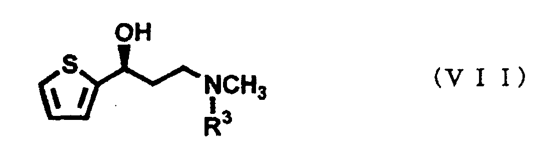 wherein R³ is as defined above, which comprises the step of reacting a ketone compound represented by the following general formula (VI): 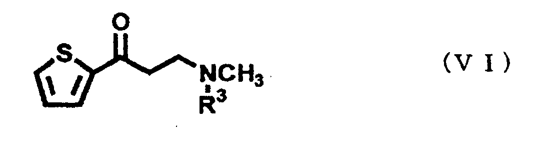 wherein R³ represents an alkyl group having 1 to 8 carbon atoms which may have a substituent or a benzyl group which may have a substituent, with the exception that R³ is a methyl group, with hydrogen in the presence of an asymmetrical hydrogenation catalyst containing a transition metal, thereby conducting asymmetrical hydrogenation of the ketone compound.

71. The process for preparing a propanolamine derivative in the form of the S-isomer according to claim 70, wherein the asymmetrical hydrogenation of the ketone compound is conducted in the presence of a base and an optically active nitrogen-containing compound, in addition to the asymmetrical hydrogenation catalyst.

72. The process for preparing a propanolamine derivative in the form of the S-isomer according to claim 71, wherein the base is any of a hydroxide, an alkoxylated compound, a mercaptized compound, a naphthylated compound and a quaternary ammonium salt of alkali metal or alkali earth metal.

73. The process for preparing a propanolamine derivative in the form of the S-isomer according to claim 71, wherein the optically active nitrogen-containing compound is an optically active amine compound.

74. The process for preparing a propanolamine derivative in the form of the S-isomer according to claim 71, wherein the optically active amine compound is (R)-1-isopropyl-2,2-di(p-methoxyphenyl)ethylenediamine.

75. The process for preparing a propanolamine derivative in the form of the S-isomer according to claim 70, wherein R³ is a benzyl group in the propanolamine derivatives represented by the general formulas (VI) and (VII).

76. The process for preparing a propanolamine derivative in the form of the S-isomer according to claim 70, wherein the asymmetrical hydrogenation catalyst is a complex of the group VIII transition metal.

77. The process for preparing a propanolamine derivative in the form of the S-isomer according to claim 76, wherein the group VIII transition metal is ruthenium.

78. The process for preparing a propanolamine derivative in the form of the S-isomer according to claim 76, wherein the asymmetrical hydrogenation catalyst has an optically active ligand.

79. The process for preparing a propanolamine derivative in the form of the S-isomer according to claim 78, wherein the optically active ligand is a phosphine ligand.

80. The process for preparing a propanolamine derivative in the form of the S-isomer according to claim 79, wherein the optically active phosphine ligand is 2,2'-bis(di-3,5-xylylphosphino)-1,1'-binaphthyl (xylBINAP).

81. A process for preparing a propanolamine derivative in the form of the S-isomer represented by the following general formula (VII): 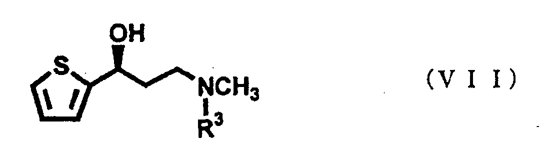 wherein R³ is as defined below, which comprises the step of obtaining a diastereomer salt of a propanolamine derivative represented by the following general formula (III): 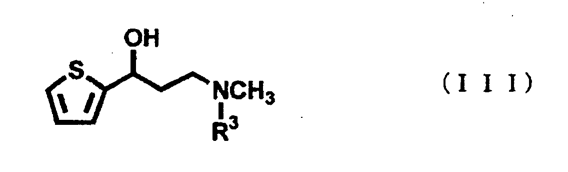 wherein R³ represents an alkyl group having 1 to 8 carbon atoms which may have a substituent or a benzyl group which may have a substituent, with the exception that R³ is a methyl group, with an optically active organic acid, and optically resolving the diastereomer salt.

82. The process for preparing a propanolamine derivative in the form of the S-isomer according to claim 81, wherein R³ is a benzyl group in the propanolamine derivatives represented by the general formulas (III) and (VII).

83. The process for preparing a propanolamine derivative in the form of the S-isomer according to claim 81, wherein the optically active organic acid is any of an optically active carboxylic acid, an optically active sulfonic acid and an optically active phosphonic acid, which is represented by the following general formula (XXI): 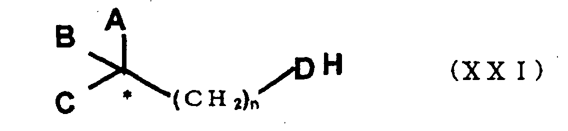 wherein D represents any of COO⁻, SO₃⁻ and PO₃H⁻, A, B, C each independently represents any of a hydrogen atom, a substituted or unsubstituted linear or branched alkyl group having 1 to 10 carbon atoms, a halogen atom, an alkoxy group, a hydroxyl group, a nitro group, a carboxyl group and a substituted or unsubstituted phenyl or naphthyl group, a substituent of the alkyl group, the phenyl group and the naphthyl group represents any of a linear or branched alkyl group having 1 to 10 carbon atoms, a halogen atom, an alkoxy group, a hydroxyl group, a nitro group, a carboxyl group and a sulfonate group, A, B, C and (CH₂)ₙ-DH each represent a different substituent, n represents 1 or 0, and the symbol * represents an asymmetric carbon.

84. The process for preparing a propanolamine derivative in the form of the S-isomer according to claim 83, wherein the optically active organic acid is an optically active mandelic acid derivative represented by the following general formula (XXII): 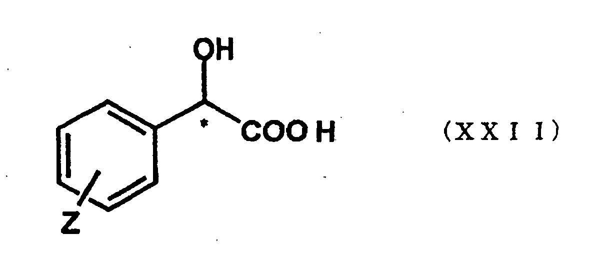 wherein Z represents any of a hydrogen atom, a linear or branched alkyl group having 1 to 10 carbon atoms, a halogen atom, an alkoxy group, a hydroxyl group, a nitro group and a benzoyl group, and the symbol * represents an asymmetric carbon.
